# NEW EUROPEAN PATENT SPECIFICATION

(11) **EP 0 116 718 B2**
(45) Date of publication and mention of the opposition decision: **08.05.1996**
(45) Mention of the grant of the patent: 16.05.1990
(21) Application number: 83112985.3
(22) Date of filing: 22.12.1983
(51) Int. Cl.: C12N 15/82, C12N 15/84, C12N 1/20, C12N 5/00, C12R 1/01, C12R 1/91

(54) **Process for the introduction of expressible genes into plant cell genomes and agrobacterium strains carrying hybrid Ti plasmid vectors useful for this process**
Verfahren zum Einbringen von expressionsfähigen Genen in Pflanzenzellgenome und hybride Ti Plasmidvektoren enthaltende Agrobacterium-Stämme verwendbar in diesem Verfahren
Procédé pour l'introduction de gènes exprimables dans les génomes de cellules de plantes et souches d'agrobactérium contenant des vecteurs plasmidiques Ti-hybrides utilisables dans ce procédé

(30) Priority: 13.01.1983 EP 83100255
(43) Date of publication of application: 29.08.1984
(60) Divisional application: 88105808.5
(73) Proprietor: Max-Planck-Gesellschaft zur Förderung der Wissenschaften e.V., D-37073 Göttingen (DE)
(72) Inventor: Zambryski, Patricia, B-9000 Gent (BE); Schell, Josef S., D-5000 Köln 30 (DE); Hernalsteens, Jean Pierre E.C., B-1150 Brussels (BE); Van Montagu, Marc Charles, B-1050 Brussels (BE); Herrera Estrella, Luis Rafael, B-9000 Gent (BE); Leemans, Jan Josef August, B-2920 Bonheiden (BE)
(74) Representative: VOSSIUS & PARTNER

(56) References cited:
- JOURNAL OF MOLECULAR AND APPLIED GENETICS, vol. 1, 1981, pages 149-164, Raven Press, New York, USA J.LEEMANS et al.: "Site-specific mutagenesis of Agrobacterium Ti plasmids and transfer of genes to plant cells"
- MOLECULAR BIOLOGY OF PLANT TUMORS, 1982, pages 537-545, Academic Press Inc.J.LEEMANS et al.: "Ti plasmids and directed genetic engineering"
- CHEMICAL ABSTRACTS, vol. 100, 1984, page 129, no. 46118f, Columbus, Ohio, USAP. ZAMBRYSKI et al.: "Ti plasmid vector for the introduction of DNA into plant cells without alteration of their normal regeneration capacity"
- NATURE, vol. 303, 19th May 1983, pages 209-213, Macmillan Journals Ltd. L. HERRERA-ESTRELLA et al.: "Expression of chimaeric genes transferred into plant cells using a Ti-plasmid-derived vector"
- THE EMBO JOURNAL, vol. 2, no. 3, 1983, pages 411-417, IRL Press Ltd., Oxford, GB.E. VAN HAUTE et al.: "Intergeneric transfer and exchange recombination of restriction fragments cloned in pBR322: a novel strategy for the reversed genetics of the Ti plasmids of Agrobacterium tumefaciens"
- NUCLEIC ACIDS RES. 13 (1985), p. 4777-4788
- EMBO JOURNAL 3 (8), 1681-89 (1984)

## Description

### Technical field of invention

This invention relates to recombinant molecules, combinations thereof processes for their preparation, and processes for their introduction into plant cells and the plant cells or plants, respectively, containing foreign DNA sequences in their genome. More particularly, the invention relates to DNA sequences to be expressed in appropriate host plant cells. The recombinant DNA molecules contemplated are characterized by sequences that code for products, e.g. amino acids or polypeptides, useful for the growth of the plant or for improving its quality as nutrient, or for the production of valuable metabolites (e.g. alkaloids or steroid precursors).

In the description the following terms are used:
*bom* site:
   region of DNA where *mob* functions specifically interact and initiate autonomous DNA transfer.
Border sequence:
   DNA sequence which contains the ends of the T-DNA.
Broad-host-range replicon:
   a DNA molecule capable of being transferred and maintained in many different host cells.
Callus tissue:
   a mass of unorganized and undifferentiated cells.
Cloning:
   the process of obtaining a population of organisms or DNA sequences derived from one such organism or sequence by asexual reproduction.
   or better:
   the process of isolating a particular organism or part thereof, and the propagation of this subfraction as a homogenous population.
Cloning vehicle:
   a plasmid, phase DNA or other DNA sequences which are able to replicate in a host cell, characterized by one or a small number of endonuclease recognition sites at which such DNA sequences may be cut in a determinable fashion without attendant loss of an essential biological function of the DNA, e.g., replication, producton of coat proteins or loss of promoter or binding sites, and which contain a marker suitable for use in the identification of transformed cells, e.g., tetracycline resistance, or ampicillin resistance. A cloning vehicle is often called vector.
Coding sequence:
   DNA sequence which determines the amino acid sequence of a polypeptide.
Cointegrate:
   the structure resulting from a single cross-over event between two circular DNA molecules.
Complementation *in trans:*
   process whereby a DNA molecule (replicon) which is not physically linked to another replicon can provide a diffusible substance which is missing and required by the other nonlinked replicon.
Conjugation:
   the process whereby DNA is transferred from bacteria of one type to another type during cell-to-cell contact.
Crossing-over:
   the process of exchange of genetic material between homologous DNA sequences.
Deletion substitution:
   removal of one DNA sequence and its replacement by a different DNA sequence.
Differentiation:
   the process whereby descendents of a cell achieve and maintain specialization of structure and function.
DNA sequence or DNA segment:
   a linear array of nucleotides connected one to the other by phosphodiester bonds between the 3' and 5' carbons of adjacent pentoses.
Double cross-over:
   the process of resolution of a cointegrate structure into two circular DNA molecules. This process is used to exchange genetic information. One of the DNA circles has two regions of homology with the target DNA through which recombination can occur; these two regions bracket a nonhomologous DNA sequence which is to be exchanged with the target DNA. If this second cross-over occurs in the same region of DNA as the first, the original DNA circles will be generated. If this second cross-over occurs in the second homologous region, genetic exchange will have occurred between the two circles.
Expression:
   the process undergone by a structural gene to produce a polypeptide. It is a combination of transcription and translation.
Expression control sequence:
   a sequence of nucleotides that controls and regulates expression of structural genes when operatively linked to those genes.
F-type plasmid:
   plasmid carrying the F (fertility) factor which allows the transfer of a copy of the plasmid to a host not carrying the F-factor.
Gene:
   a DNA sequence composed of two parts, (1) the coding sequence for the gene product, and (2) the sequences in the promoter region which control whether or not the gene will be expressed.
Genome:
   the entire DNA of a cell or a virus. In includes *inter alia* the structural genes coding for the polypeptide(s), as well as operator, promoter and ribosome binding and interaction sequences, including sequences such as the Shine-Dalgarno sequences.
Genotype:
   the sum total of the genetic information contained in an organism.
Homologous recombination:
   recombination between two regions of DNA which contain homologous sequences.
I-type plasmid:
   a group of autotransferable plasmids of a different incompatibility group than F.
Incompatibility:
   when two DNA molecules are not capable of coexisting in the same cell in the absence of selective pressure.
Insertion:
   addition of a DNA sequence within the DNA sequence of another molecule.
Leader sequence:
   the region of an mRNA molecule extending from the 5' end to the beginning of the first structural gene; it also includes sites important to initiate translation of the coding sequence of the structural gene.
Meiosis:
   two successive divisions that reduce the starting number of 4 n chromosomes to 1 n in each of four product cells. This process is important in sexual reproduction.
*mob* (mobilization functions):
   a set of products which promote DNA transfer only in combination with *tra* functions. *Mob* can promote transfer of plasmids containing a *bom* site.
Mobilization:
   the process whereby one DNA molecule which is not able to transfer to another cell is helped to transfer by another DNA molecule.
Mobilization helper plasmid:
   a plasmid capable of providing diffusible products which another plasmid lacks for transfer to another host cell.
Nonconjugative recombinant plasmid:
   a DNA molecule which is not capable of being transferred by itself from its host cell to another host cell during cell-to-cell contact. For transfer it will need further functions supplied by other DNA, e.g. by (a) helper plasmid(s).
Nucleotide:
   a monomeric unit of DNA or RNA consisting of a sugar moiety (pentose), a phosphate, and a nitrogenous heterocyclic base. The base is linked to the sugar moiety via a glycosidic bond (1' carbon of the pentose) and that combination of base and sugar and is a nucleoside. The base characterizes the nucleotide. The four DNA bases are adenine ("A"), guanine ("G"), cytosine ("C"), and thymine ("T"). The four RNA bases are A, G, C, and uracil ("U").
Phenotype:
   the observable characteristics of an individual resulting from the interaction between the genotype and the environment in which development occurs.
Plasmid:
   a nonchromosomal double-stranded DNA sequence comprising an intact "replicon" such that the plasmid is replicated in a host cell. When the plasmid is placed within a unicellular organism, the characteristics of that organism are changed or transformed as a result of the DNA of the plasmid. For example, a plasmid carrying the gene for tetracycline resistance (Tc^{R}) transforms a cell previously sensitive to tetracycline into one which is resistant to it. A cell transformed by a plasmid is called "transformant".
Polypeptide:
   a linear series of amino acids connected one to the other by peptide bonds between the α-amino and carboxy groups of adjacent amino acids.
Promoter region:
   DNA sequences upstream to the start of the coding sequence which regulate transcription of the gene.
Promoter sequence:
   sequence at which RNA polymerase binds and promotes the faithful transcription of downstream sequences.
Recombinant DNA molecule or hybrid DNA:
   a hybrid DNA sequence comprising at least two nucleotide sequences, the first sequence not normally being found together in nature with the second.
Recombination:
   the creation of a new association of DNA molecules or parts of DNA molecules.
Region of homology:
   a region of DNA which shares the same nucleotide sequence as that found in a region of another DNA.
Replicon:
   a self-replicating genetic element possessing a site for the initiation of DNA replication and genes specifying the necessary functions for controlling replication.
Restriction fragment:
   a DNA molecule resulting from double-stranded cleavage by an enzyme which recognizes a specific target DNA sequence.
RNA polymerase:
   enzyme which results in the transcription of DNA into RNA.
Selectable marker gene:
   a DNA sequence which, when expressed, gives that cell a growth advantage over cells which do not contain that DNA sequence, when all cells are in another growth medium which can distinguish the two types of cells. Commonly used selectable marker genes are those which encode resistance to antibiotics.
Single cross-over:
   the process of recombining two circular DNA molecules to form a cointegrate larger circle.
Structural gene:
   a gene which codes for a polypeptide.
T-DNA:
   portion of the Ti plasmid as it is found stably integrated into the plant cell genome.
T-region:
   portion of the Ti plasmid which contains the DNA sequences which are transferred to the plant cell genome.
Ti plasmid:
   large plasmids found in strains of *Agrobacterium tumefaciens* containing the genetic information for tumor (crown gall) induction on susceptible plants.
TL-DNA and TR-DNA:
   octopine crown gall tumor cells can contain two T-DNA sequences, a left T-DNA, i.e. TL-DNA, and a right TR-DNA. TL-DNA contains sequences also found in common with T-DNA of nopaline tumor cells whereas TR-DNA does not.
*tra* (transfer functions):
   both plasmid-encoded diffusible products and sites of action utilized during DNA transfer between cells, e.g. products required to make a bridge between two cells and the site at which DNA transfer is initiated.
Transcription:
   the process of producing mRNA from a structural gene
   or:
   the process involving base paring whereby the genetic information contained in DNA is used to order a complementary sequence of bases in an RNA chain.
Transformation:
   genetic modification induced by the incorporation of exogenous DNA into the DNA complement of a cell.
Translation:
   the process of producing a polypeptide from mRNA
   or:
   the process whereby the genetic information present in an mRNA molecule directs the order of specific amino acids during the synthesis of a polypeptide.
Undifferentiated phenotype:
   a homologous appearance of cells in a tissue without any specialized parts.
Vector:
   a DNA molecule designed for transfer between different host cells.

### Background art

The development of recombinant DNA techniques has made the genetic engineering of microorganisms a challenging prospect. These techniques might be extended to multicellular eukaryotes, if complete organisms could be regenerated from single somatic cells. The cells of some higher plants exhibit excellent regeneration capacities and, therefore, are good materials for the genetic engineering of higher organisms.

A major problem for the genetic engineering of plants is the availability of a system for the introduction of exogenous (foreign) DNA into the plant genome. Such a system is provided by the tumor-inducing (Ti) plasmids carried by the Gram-negative soil bacterium *Agrobacterium tumefaciens.* This organism has been shown to cause a neoplastic transformation, called "crown gall", of wounded tissue of a very wide range of dicotyledonous plants. The proliferating neoplasms synthesize novel, Ti-specified metalolites, called opines. The molecular basis of this transformation is the transfer and stable integration of a well-defined T-DNA (transferred DNA) fragment of the Ti plasmid in the plant cell genome. In other words, crown gall tumors contain in their chromosomal DNA a DNA segment called the T-DNA which is homologous to DNA sequences in the Ti plasmid used to induce the tumor line. In all cases, this T-DNA corresponds to, and is colinear with, a continuous stretch of Ti plasmid DNA which is therefore, called the T-region.

Ti plasmids are classified according to the type of opine synthesized in crown gall cells. *Agrobacterium* strains which induce the synthesis of nopaline [N-α-(1,3-dicarboxypropyl)-L-arginine] in crown gall cells are called nopaline strains, and strains which induce the synthesis of octopine [N-α-(N-1-carboxyethyl)-L-arginine] are called octopine strains. These are the most commonly used *Agrobacterium* strains*.*

The use of T-DNA as a vector for plant genetic engineering was demonstrated in a model experiment in which the 14 kb bacterial transposon Tn7 was inserted *in vivo* near the right border of the T-DNA from the Ti plasmid of the strain *Agrobacterium* T37. Nopaline synthesis was eliminated in the tumors incited by agrobacteria carrying this Ti plasmid. Furthermore, Southern blotting hybridizations revealed that the entire Tn7 was present in the chromosomal DNA of these tumors as part of an otherwise normal T-DNA sequence (Hernalsteens *et al., Nature* 287 (1980), 654-656; Holsters *et al., Mol. Gen. Genet.* 185 (283-289). Thus, the introduction of this 14 kb DNA fragment into the 23 kb T-DNA has not altered the latter's ability to be transferred to the plant cell genome.

The borders of the T-DNA from the Ti plasmid of the nopaline strain *Agrobacterium* T37 have been very precisely determined. It is only a portion, roughly 23 kb, of the entire nopaline Ti plasmid. Furthermore, the borders of the T-DNA are known; the nucleotide sequences which define the borders of the T-DNA have been determined and compared with the same region of the nopaline Ti plasmid (Zambryski *et al., Science* 209 (1980), 1385-1391; Zambryski *et al., J. Mol. Appl. Genet.* 1 (1982), 361-370). The borders of the T-region are most probably involved in the integration of the T-DNA into the plant cell genome.

Knowledge of the T-DNA sequences which define the borders of the transferred DNA is a basic requirement for the use of the Ti plasmid as a vector for DNA transfer to plant cells. Thus, foreign DNA can be inserted within these borders to ensure its transfer to the plant cell genome. In addition, if one expects to utilize this system it is important that the transformed plant cells are normal rather than tumorous in their growth properties. To produce normal cells after T-DNA transfer requires knowledge of the functions encoded by the T-DNA itself. Thus, the T-region of the Ti plasmid has been subjected to intense genetic analysis to determine which regions are responsible for the tumor phenotype.

The T-DNA encodes functions which are responsible for the crown gall phenotype. The genes have been localized to specific regions of the T-DNA (Leemans *et al.*, *EMBO J.* 1 (1982), 147-152; Willmitzer *et al.*, *EMBO J.* 1 (1982), 139-146). In general, there are at least 4 genes which control the undifferentiated phenotype of tumor callus tissue. Mutants in these genes can either lead to transformed tissues which appears shoot-like or root-like. The latter results are especially important if one hopes to transfer DNA to plants for expression in normal plant tissue rather than tumor tissue.

Recently, a mutant Ti plasmid was found to induce transformed shoots which were capable for regenerating into completely normal plants. These plants were fertile and even transmitted T-DNA specific sequences through meiosis, i.e. progeny plants still contained T-DNA-specific sequences (Otten *et al, Mol. Gen. Genet.* 183 (1981), 209-213). However, the transformed plant tissue contained in its chromosomal DNA a T-DNA which was very much reduced in size due to the generation of a large deletion which removed the region of the T-DNA controlling the tumor phenotype. It is not known whether the deletion occurred during the initial transformation event or as a subsequent event leading to shoot formation.

The Ti plasmids are large (200 kb) and many genes located at different sites on the Ti plasmid are involved in the transformation of plant cells. Therefore, it is not possible to construct a small Ti plasmid-derived cloning vector with unique endonuclease recognition sites at appropriate locations within the T-region, and possessing all functions essential for T-DNA transfer and stable incorporation into the plant cell genome. One known way to introduce a chosen DNA fragment into specific restriction enzyme cleavage sites of the T-region of a Ti plasmid has been to construct cloning plasmids which are able to replicate in *Agrobacterium* as well as in *Escherichia coli,* and which contain a chosen restriction fragment of the T-DNA. Such cloning plasmids were denoted "intermediate vectors". Such intermediate vectors were used to analyze the functions encoded by the T-region (Leemans *et al., J. Mol. Appl. Genet.* 1 (1981), 149-164).

### Disclosure of the invention

The present invention relates to a process for the introduction of expressible genes into plant cell genomes. It is one of the objects of the present invention to provide vector combinations consisting of modified acceptor Ti plasmids and intermediate cloning vectors which allow the introduction of any gene(s) of interest into these genomes. The gene(s) of interest to be introduced is (are) contained within a novel intermediate cloning vector carrying a region of homology with a corresponding region in the acceptor Ti plasmid. The intermediate cloning vectors are a further object of this invention.

The introduction of the gene(s) of interest into the acceptor Ti plasmids is achieved by a single cross-over event which occurs within the two homologous DNA segments of the acceptor Ti plasmid harbored in *Agrobacterium* and the intermediate cloning vector. The intermediate cloning vector is mobilized from *Escherichia coli* where it is propagated to *Agrobacterium* using helper plasmids. Such helper plasmids and their functions for mobilization are known (Finnegan *et al., Mol. Gen. Genet.* 185 (1982), 344-351; *Figurski* et al., Proc. Natl. Acad. Sci. USA 76 (1979), Ditta et al., Proc. Natl. Acad. Sci. USA 77 (1980), 7347). The result of the single cross-over event in *Agrobacterium* is a hybrid Ti plasmid vector. Such hybrid Ti plasmid vectors are a further object of the invention.

The resulting hybrid plasmid vectors harbored in *Agrobacterium* (hereinafter called vector composition) can be used directly to infect plant cells which are subsequently screened for the expression of the product(s) of the gene(s) of interest. This process for preparing transformed plant cells by infection of plant cells with the vector composition, and the transformed plant cells, as well as plants generated therefrom are further objects of this invention. This strategy is applicable to any of the plant-transferable plasmids of *Agrobacterium.*

### Description of drawings

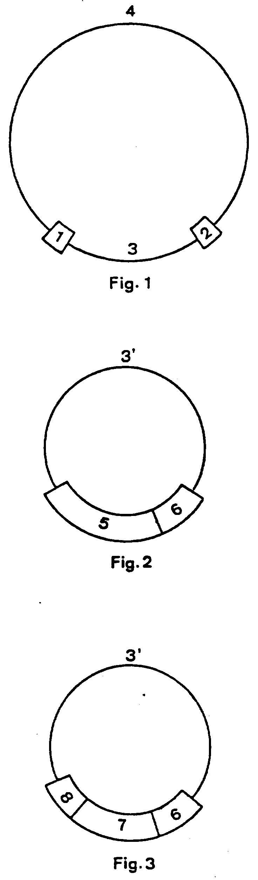

Fig. 1 illustrates one embodiment of an acceptor Ti plasmid of the invention which is the result of a deletion of the internal portion of the T-region, except for the border sequences (1) and (2). The border sequences are essential for the integration of the T-region into a plant cell genome. The region (3) between border sequences (1) and (2) is the DNA segment which will be transferred to the plant. The acceptor Ti plasmid contains a DNA segment (3) with a DNA sequence which is homologous with at least a part of a DNA sequence in an intermediate cloning vector permitting integration of the intermediate cloning vector via a single cross-over event. The Ti plasmid region (4) codes for functions which are essential for the transfer by *Agrobacterium* of the T-region into plant cell genomes. This region has been designated as *vir*-region.

Fig. 2 illustrates an intermediate cloning vector of the invention to be inserted by a single cross-over event into the acceptor Ti plasmid of Fig. 1. It contains a cloning vehicle DNA segment (3') with a DNA sequence which is homologous with at least part of the DNA segment (3) of the acceptor Ti plasmid permitting the desired single cross-over event. Moreover, the intermediate cloning vector contains a gene or group of genes (5) of interest having its natural promoter sequence. In general plant genes can be used in this construction as they are more likely to be expressed. However, in principle any natural gene of interest can be inserted. The intermediate cloning vector may also contain a selectable marker gene (6). This gene should contain a promoter sequence which permits expression of the gene in plant cells. Plant cells containing this marker gene should have a selective growth advantage over cells without this gene; in this way plant cells which have been transformed by DNA containing this marker gene can be distinguished from untransformed plant cells.

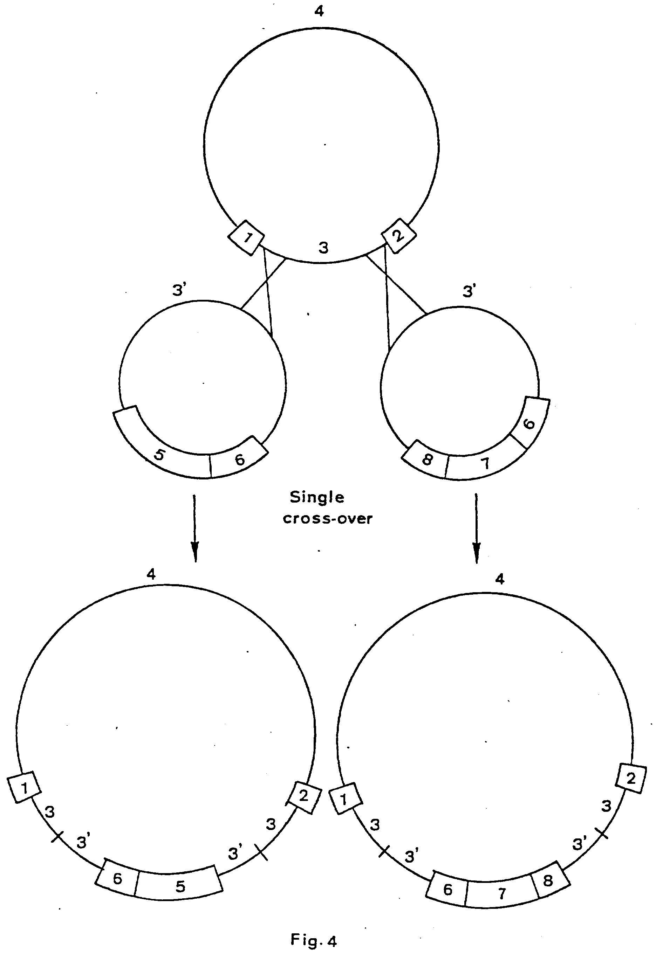

Fig. 3 illustrates another embodiment of an intermediate cloning vector of the invention which is similar to the intermediate cloning vector of Fig. 2, and is to be inserted by a single cross-over event into the acceptor Ti plasmid of Fig. 1. It contains the cloning vehicle DNA segment (3'), an exogenous promoter sequence (8) allowing regulated expression of the gene(s) of interest (7), and, if desired, a marker gene (6).

Fig. 4 schematically illustrates the construction of hybrid Ti plasmid vectors of the invention from the acceptor Ti plasmid of Fig. 1, and the intermediate cloning vectors of Figs. 2 and 3 by a single cross-over event.

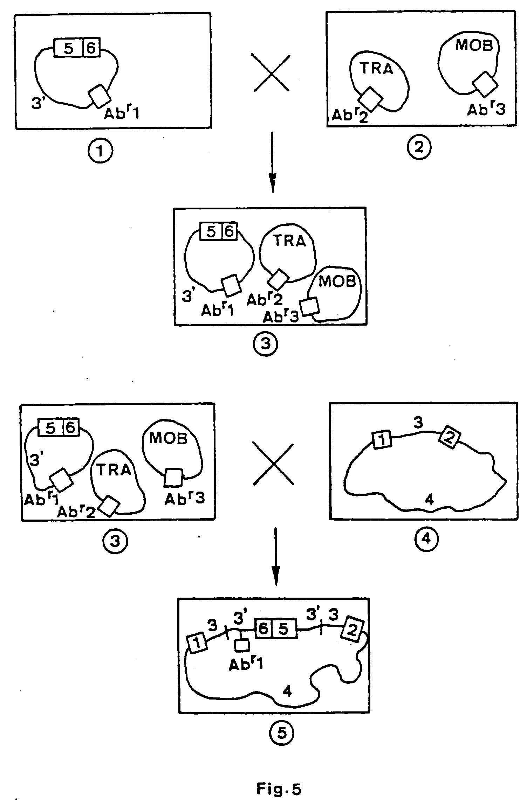

Fig. 5 outlines the steps involved in the genetic transfer of an intermediate cloning vector from *E. coli* to *Agrobacterium* containing an acceptor Ti plasmid. The first step is the conjugation of the *E. coli* strain (1) containing the intermediate cloning vector to another *E. coli* strain (2) which contains two helper plasmids for the later conjugation to *Agrobacterium..* One helper plasmid contains DNA sequences important for plasmid transfer *(tra)* and the other helper plasmid contains sequences which are important for the mobilization *(mob).* When these helper plasmids are introduced by conjugation into *E. coli* strain (1), the intermediate cloning vector contained therein will be capable of being transferred to other bacterial strains. The *tra* and *mob* helper plasmids also contain antibiotic resistance markers Ab^{r2} and Ab^{r3} which are different from those found in the intermediate cloning vector (Ab^{r1}). Thus, the presence of all the plasmids can be monitored on selective media. A mobilizing strain (3) is obtained. This mobilizing strain (3) is conjugated to an *A. tumefaciens* strain (4) which contains the acceptor Ti plasmid of Fig. 1 followed by selection for antibiotic resistance marker(s) of the intermediate cloning vector. As the intermediate cloning vector cannot replicate in *Agrobacterium,* it can only be maintained if it has formed a cointegrate with the recipient acceptor Ti plasmid; this cointegrate structure in *Agrobacterium* (5) is the final hybrid Ti plasmid used to transfer DNA into plant cell genomes.

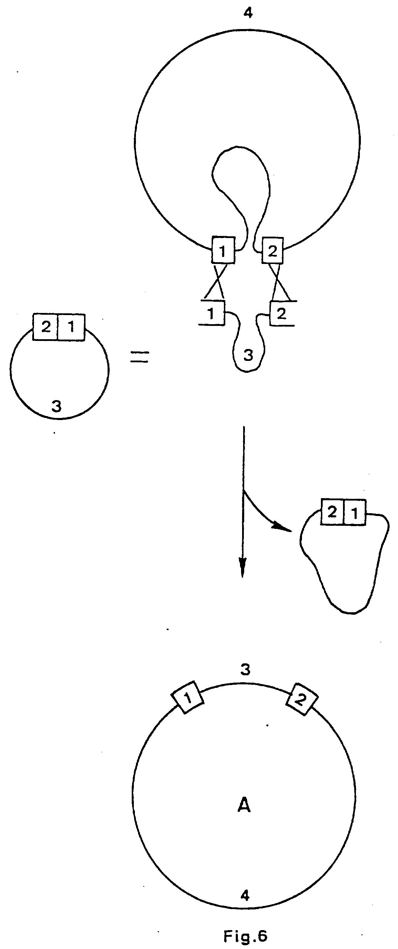

Fig. 6 illustrates the construction of a model acceptor Ti plasmid (A) which is analogous to that shown in Fig. 1. Here, a double cross-over event occurs between a Ti plasmid and another plasmid containing a DNA sequence which is to replace a portion of the original Ti plasmid. More specifically, the smaller plasmid contains the border sequences of the T-region (1; 2) in a cloning vehicle (3). A double cross-over results in the deletion of the internal portion T of the T-region and its replacement by the cloning vehicle. The acceptor Ti plasmid (A) obtained is capable of transferring DNA contained between the border sequences (1; 2) into plant cell genomes. The resulting transformed plant DNA will not produce tumorous crown gall tissue as the genes controlling neoplastic growth are deleted in the Ti plasmid (A). Ti plasmid (A) is a very generalized acceptor Ti plasmid for any intermediate cloning vector with homology with cloning vehicle (3). The cloning vehicle (3) may be a conventional plasmid, such as pBR322 (or its derivatives).

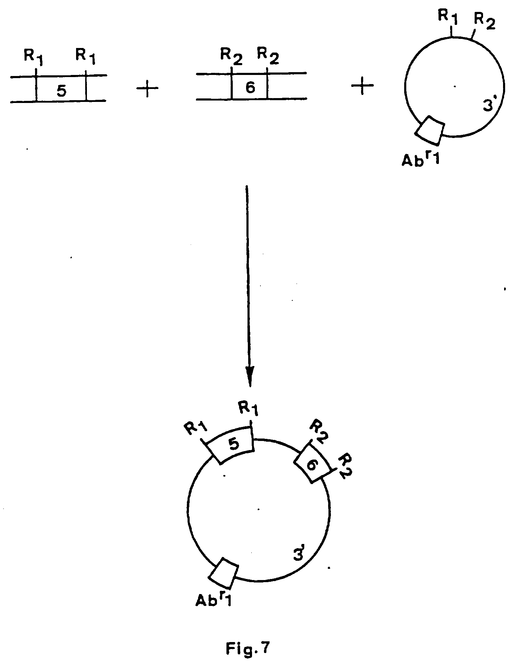

Fig. 7 illustrates schematically the steps leading to the construction of an intermediate cloning vector in E. *coli* host cells. A gene of interest (5) bracketed by restriction endonuclease site R1 and a selectable marker gene (6) bracketed by restriction endonuclease site R2 are inserted into a cloning vehicle (3') containing single restriction sites for the enzymes R1 and R2. All three molecules are digested with restriction enzymes R1 and/ or R2 and ligated together using DNA ligase to form the intermediate cloning vector. The cloning vehicle 3' must also contain additional DNA sequences which code for antibiotic resistance (Ab^{r1}) to use as a selectable marker for bacterial genetics. The gene of interest (5) may be under the control of its natural promoter or of an exogenous promoter as outlined in Figs. 2 and 3.

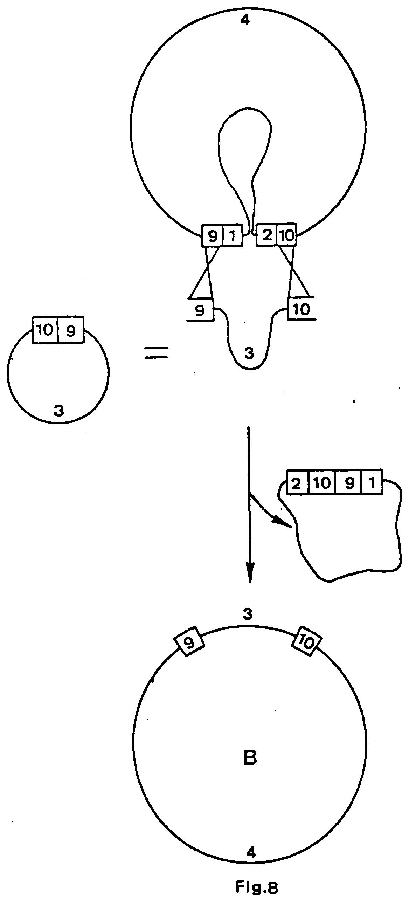

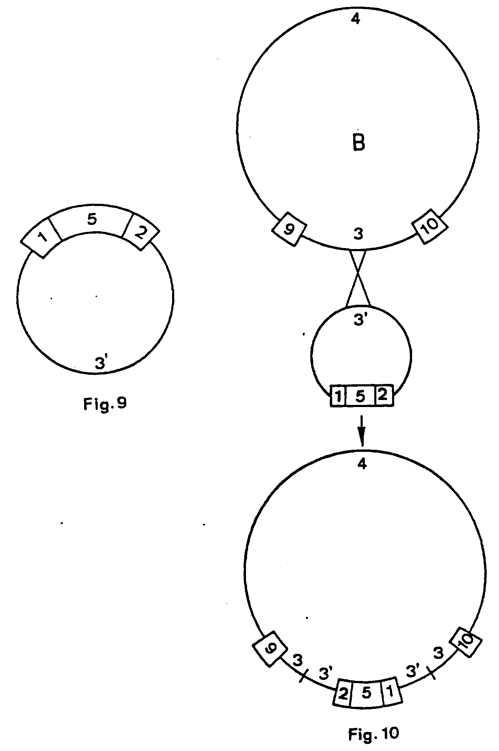

Fig. 8 illustrates the construction of another embodiment of an acceptor Ti plasmid (B) of the invention. Here, a double cross-over event occurs between a Ti plasmid and a cloning vehicle (3) which contains DNA sequences (9) and (10) which are homologous to Ti sequences just outside the border sequences (1) and (2) respectively. The double cross-over event results in the deletion of the entire T-region T including the border sequences (1) and (2) and its replacement with the cloning vehicle (3). Ti plasmid (B) is an acceptor for intermediate cloning vectors which contain the gene of interest cloned between the border sequences (1) and (2); (see Fig. 9).

Fig. 9 illustrates an intermediate cloning vector of the invention to be inserted by a single cross-over event into the acceptor Ti plasmid (B) of Fig. 8. It contains the border sequences (1) and (2) which flank the gene(s) of interest (5). It also contains a cloning vehicle sequence (3') which is at least partially homologous to the cloning vehicle sequence (3) in the acceptor Ti plasmid (B) to allow homologous recombination between the two plasmids.

Fig. 10 schematically illustrates the construction of hybrid Ti plasmid vectors of the invention from the acceptor Ti plasmid (B) of Fig. 8 and the corresponding intermediate cloning vector of Fig. 9. A single cross-over event introduces the intermediate cloning vector of Fig. 9 into the acceptor Ti plasmid (B) of Fig. 8.

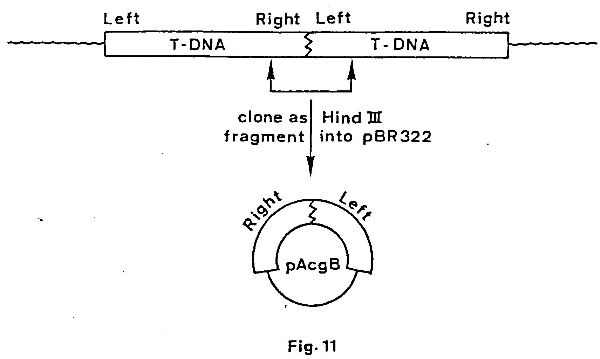

The following Figures 11 to 20 more specifically illustrate the invention.

Fig. 11 illustrates the insertion of the 5.2 kb *Hin*dlll fragment AcgB into pBR322 (see also Zambryski *et al., Science* 209 (1980), 1385-1391). This fragment AcgB contains the right and left border regions of the nopaline Ti plasmid T-DNA. This clone pAcgB is used in the construction of acceptor plasmid pGV3850, an "A-like" acceptor plasmid as shown in Fig. 6. It is obvious to those skilled in the art that a clone analogous to clone pAcgB can be obtained by using the cloned restriction fragments which contain the left and right border region of a wild-type Ti plasmid.

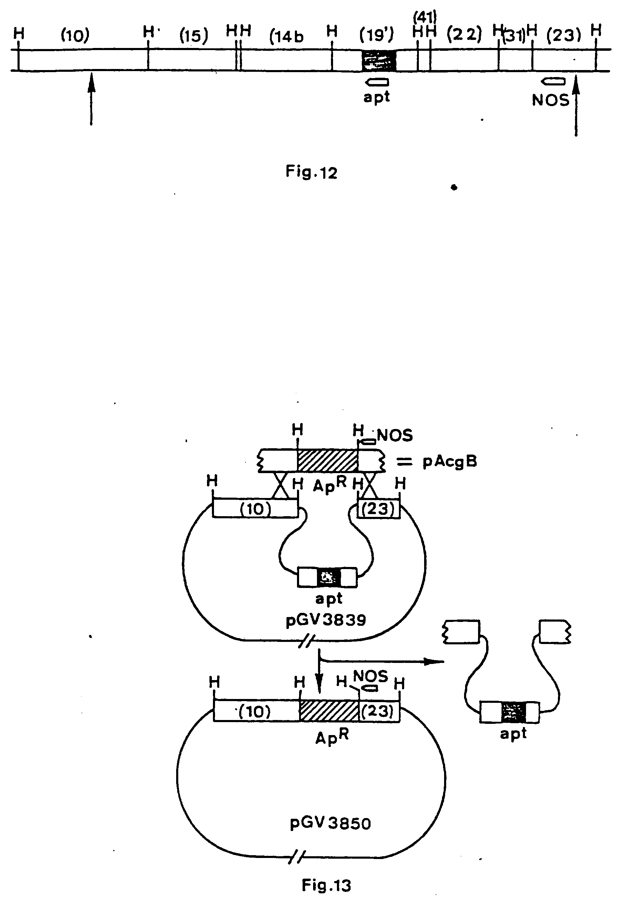

Fig. 12 illustrates the T-region of nopaline Ti plasmid pGV3839. The *Hin*dlll restriction endonuclease sites are indicated as (H). Mutated *Hin*dlll fragment 19 is indicated (19'). The acetylphosphotransferase gene providing kanamycin or neomycin resistance is indicated as *apt* and is located in the black box area. The borders of the T-region are indicated by arrows. The nopaline synthase gene is indicated by *nos.* The numbers refer to the sizes of the restriction fragments according to Depicker *et al., Plasmid* 3 (1980), 193-211. The construction of the Ti plasmid pGV3838 can be taken from Example 1, and the two references cited.

Fig. 13 illustrates the construction of acceptor Ti plasmid pGV3850. The plasmid pBR322-pAcgB (Fig. 11) is depicted in a linearized form. The pBR322 sequences are indicated by the crosshatched area and the ampicillin resistance gene of pBR322 by Ap^{R}. Part of the T-region of pGV3839 which is shown in Fig. 12 is shown here; the *Hin*dlll fragments (10) and (23) involved in homologous recombination with pAcgB and the *apt* gene are indicated. Double cross-over events lead to the construction of pGV3850 and another replicon containing the *apt* gene which is lost.

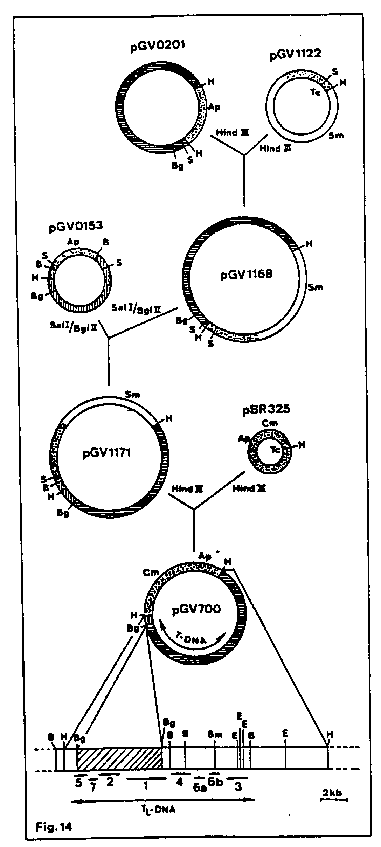

Fig. 14 illustrates schematically the construction of intermediate cloning vector pGV700 which is given in detail in Example 2. The following abbreviations are used to indicate restriction endonuclease sites: B, *Bam*HI; Bg, *BG/*II; E, *Eco*RI; H, *Hin*dIII; S, *Sa/*I; Sm, *Sma*l. The following abbreviations are used to indicate antibiotic resistance; Ap, ampicillin; Cm, chloramphenicol; Sm, streptomycin; Tc, tetracycline. The numbers on the bottom of the figure which refer to TL-DNA indicate the RNA transcripts of this region (Willmitzer *et al.*, *EMBO J.* 1 (1982), 139-146).

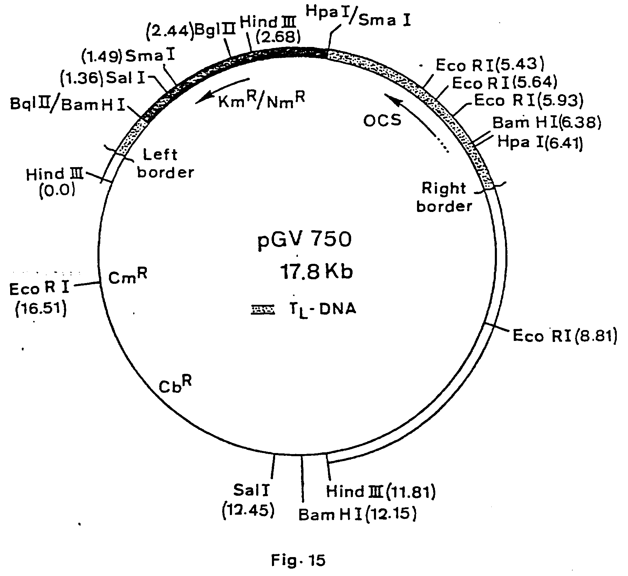

Fig. 15 illustrates the structure of the intermediate cloning vector pGV750. Its construction is described in Example 2. The restriction endonuclease sites are indicated with their relative locations given in numbers as kilobase pairs (kb). *Pst*l sites are not indicated but there are 3 in the Km^{R}/Nm^{R} region, and one in the Cb^{R} gene. The right and left borders are also indicated. The *Bg*/II/*Bam*HI and *Hpa*l/*Sma*l sites used in the construction of pGV750 are indicated but are not present in pGV750. The shaded area corresponds to TL-DNA, the dark area to the Km^{R}/Nm^{R} region, the white area to contiguous Ti plasmid sequences, and the line to the cloning vehicle pBR325. Other abbreviations used are: Ocs, octopine synthase; Cm^{R}, chloramphenicol resistance; Cb^{R}, carbenicillin (analogous to ampicillin) resistance; and Km^{R}/Nm^{R}, kanamycin resistance/neomycin resistance.

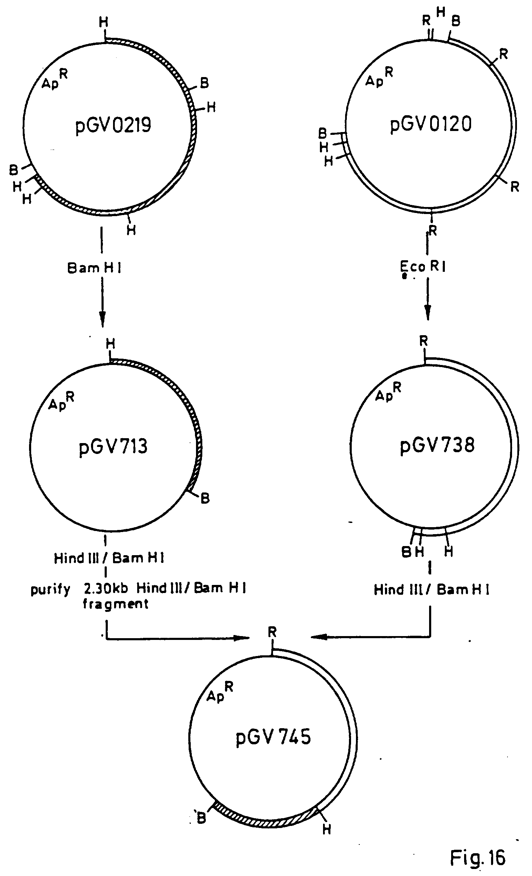

Fig. 16 illustrates the construction of the intermediate vector pGV745 described in detail in Example 3. pGV745 is used in the construction of the acceptor plasmid pGV2260, a "B-like" acceptor plasmid shown in Figure 8. Restriction endonuclease sites are indicated as follows: B, *Bam*HI; H, *Hin*dIII; R, *Eco*RI*.* The ampicillin resistance gene is indicated by Ap^{R}. The crosshatched region indicates DNA homologous to the left side of the T-DNA region of the octopine Ti plasmid and the white region indicates DNA homologous to the right side of the T-DNA region of the octopine Ti plasmid; the physical location and description of the starting plasmids pGV0219 and pGV0120 can be found in De Vos *et al., Plasmid* 6 (1981), 249-253.

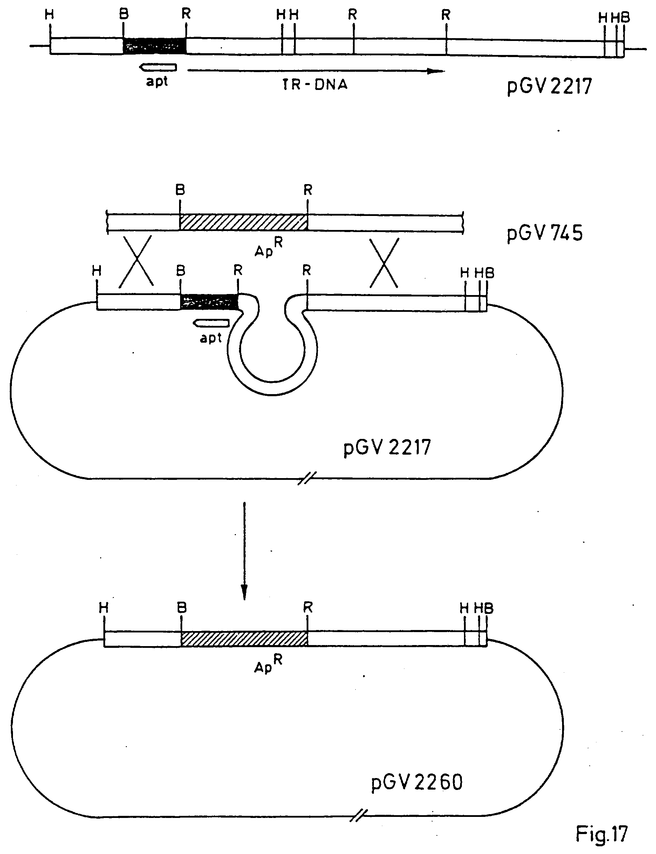

Fig. 17 illustrates the construction of the acceptor plasmid pGV2260. The deletion substitution in pGV2217 is indicated as a black box containing the acetyl phosphotransferase gene (indicated *apt)* which provides resistance to neomycin and kanamycin. The intermediate vector pGV745 (Fig. 16) is depicted in linearized form; it has been opened at the *Hin*dlll site of pGV745 shown in Fig. 16. The pBR322 sequence is indicated as a crosshatched section and the ampicillin resistance gene by Ap^{R}. Double cross-over events lead to the construction of pGV2260 and the loss of the *apt* gene. Restriction endonuclease sites are indicated as follows: B, *Bam*HI; H, *Hin*dIII; R, *Eco*RI*.*

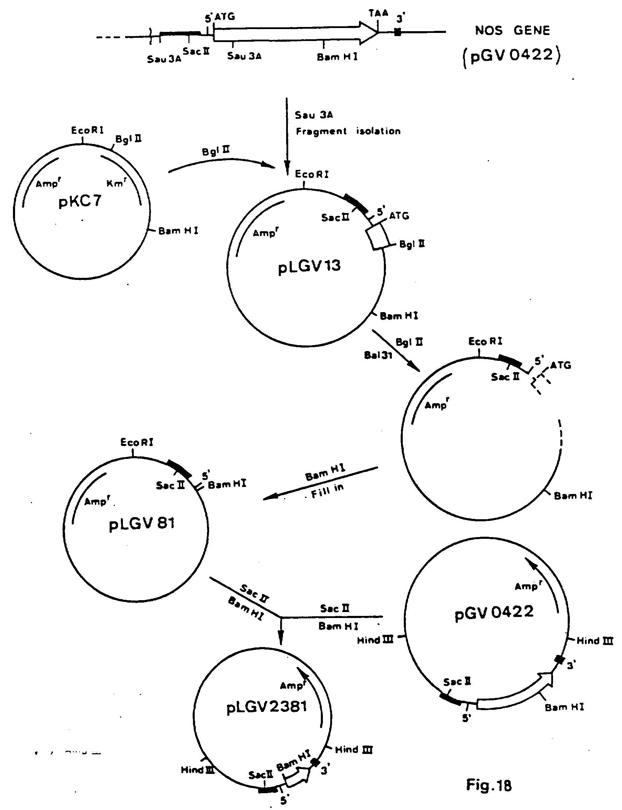

Fig. 18 illustrates the construction of a plasmid pLGV2381 for the expression of genes downstream of the promoter of the nopaline synthase gene *(nos).* The 5' and 3' refer to the start and stop of transcription, and the ATG and TAA refer to the codons used to start and stop translation. The heavy black line indicates the *nos* promoter region, and the white area indicates the *nos* coding region. AP^{R} denotes ampicillin resistance, and Km^{R} kanamycin resistance.

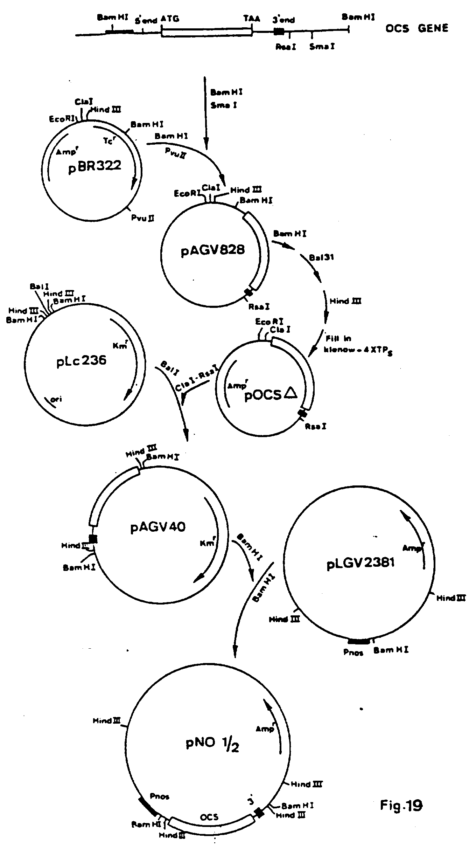

Fig. 19 illustrates the insertion of the plasmid pAGV10 containing the complete octopine synthase *(ocs)* coding sequence in plasmid pLGV2381 (see also Fig. 18) behind the *nos* promoter resulting in plasmid pNO 1/2. The heavy black lines refer to the promoter regions and the white area to the *ocs* coding region. Other notations are as in Fig. 18.

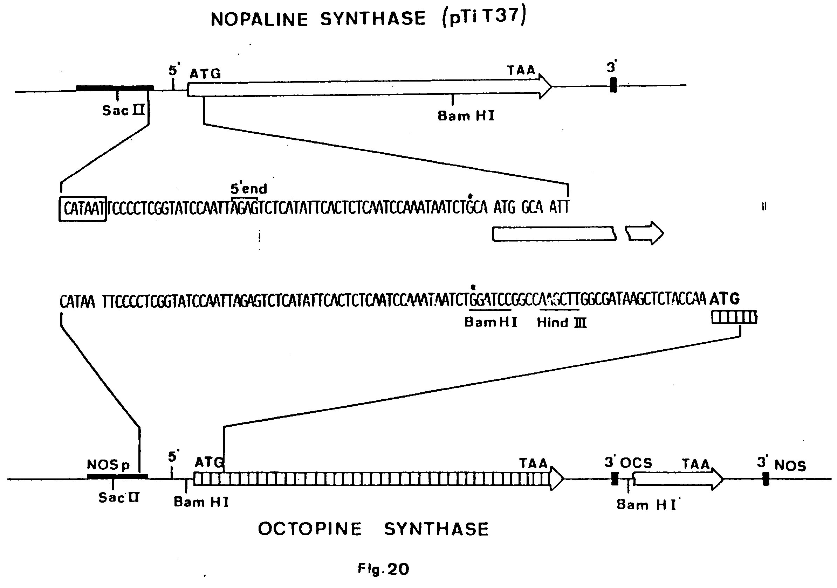

Fig. 20 illustrates the nucleotide sequences around the promoter region of the nopaline synthase *(nos)* gene and the nucleotide sequences around the same region following fusion with the coding region of the octopine synthase gene. The point of fusion is indicated by an asterisk (*). Several restriction endonuclease sites are indicated, *Bam*HI, *Hin*dIII, and *Sac*II*.* The 5' and 3' refer to the start and stop of transcription. The ATG refers to the first codon used in translation and the TAA refers to the stop codon used in translation. The large arrows indicate the coding regions of the nopaline gene in white and the octopine gene in stripes.

### Detailed description of the invention

Referring to Fig. 1, we have shown therein a simplified diagram of an acceptor Ti plasmid. This acceptor Ti plasmid contains the two border sequences (1; 2) or regions of the wild-type tumor-inducing (Ti) plasmid. The border sequences are essential for the integration of the T-region of Ti plasmids into a plant cell genome. In other words, they are essential for the integration of any DNA sequence (3) or T-region into a plant cell genome located between these sequences.

The DNA sequence (3) of the acceptor Ti plasmid contains a DNA segment which is homologous with at least a part of a DNA sequence (3') of an intermediate cloning vector illustrated in Figs. 2 and 3.

This homology is necessary for co-integration by a single cross-over event (homologous recombination) of the intermediate cloning vector with the acceptor Ti plasmid. The frequency of obtaining cointegrates is determined essentially by the length of the region of homology. In orderto effect a homologous recombination event at a good frequency, regions of 1 to 4 kb are normally used (Leemans *et al., J. Mol. Appl. Genet.* 1 (1981), 149-164).

The acceptor Ti plasmid furthermore contains sequences (4) which are essential for the transfer by *Agrobacterium* of the T-region of the Ti plasmids into plant cell genomes.

The construction of such acceptor Ti plasmids and their cointegration with intermediate cloning vectors illustrated in Figs. 2 and 3 will be described later in connection with the discussion of Fig. 4.

In Figs. 2 and 3 we have shown simplified diagrams of intermediate cloning vectors for the cloning of any prokaryotic or eukaryotic gene(s) of interest to be expressed, i.e. transcribed under the control of a promoter and translated in plant cells. These intermediate cloning vectors contain a DNA segment (3') from a cloning vehicle containing a DNA sequence which is homologous with at least a part of the DNA segment (3) of the acceptor Ti plasmid thus permitting a single cross-over event. Moreover, the intermediate cloning vectors contain at least one gene of interest (5; 7) which contains either its natural or an exogenous promoter sequence. The promoter sequence allows the expression of the inserted gene sequence(s). The use of an exogenous promoter sequence (tailored promoter) may be useful for directing the expression of the inserted gene(s) in a regulated fashion.

Examples of different types of regulation include the following: (i) tissue-specific expression, i.e. leaves, roots, stem, flowers; (ii) level of expression, i.e. high or low; and (iii) inducible expression, i.e. by temperature, light, or added chemical factors.

Examples of genes of interest for the intermediate cloning vectors are: DNA fragments or sequences with the genetic information controlling the synthesis of products such as amino acids or sugars to modify the nutritive or growth potential of a plant; or products which provide protection against external pathogenic agents such as resistance to disease organisms or stressful environmental factors: or products which provide information on basic plant processes which are to be modified by genetic engineering techniques.

Figures 2 and 3 each show intermediate cloning vectors which may contain selectable marker genes (6). Examples of selectable marker genes are those encoding resistance to antibiotics or toxic analogs (for example, amino acid analogs), or genes which can supply a defect in the recipient host cell.

Figure 4 illustrates the structures involved in the construction of hybrid Ti plasmid vectors and Figure 5 describes the actual conjugation steps involved in the isolation of *Agrobacterium* carrying the hybrid Ti plasmid vectors. Since the intermediate cloning vector is constructed in *E. coli* these steps are necessary to transfer the intermediate cloning vector to the acceptor Ti plasmid in *Agrobacterium.*

The known transfer process which was used to prepare modified Ti plasmids in which a portion of the T-region was replaced by an altered sequence involves many steps. Normally, most DNA recombinant manipulations are done in specially designed cloning vehicles such as pBR322 (Bolivar, *Gene* 2 (1977), 75-93). However, these cloning vehicles cannot transfer on their own to *Agrobacterium.* In the known process this problem is solved by:
a) replacing the pBR cloning vehicle sequence by another wide-host-range cloning vehicle, such as the mini-Sa plasmid (Leemans *et al., Gene* 19 (1982), 361-364), capable of also repliacting in *Agrobacterium.* The manipulations are effected in *E. coli.* An intermediate cloning vector is obtained.
b) Conjugation of the *E. coli* strain carrying the intermediate cloning vector containing the DNA of interest with another *E. coli* strain carrying a helper plasmid which cannot replicate in *Agrobacterium* but which can mediate transfer of itself and other DNAs to *Agrobacterium.*
c) Conjugation of *E. coli* obtained in step (b) with *Agrobacterium* containing a Ti plasmid. The helper plasmid is lost.
d) Since the intermediate cloning vector is capable of replicating and existing in *Agrobacterium* as a separate replicon, the conjugants obtained in step (c) are a mixture of cells containing either the cointegrate between the intermediate cloning vector and the Ti plasmid or other cells containing the intermediate cloning vector and the Ti plasmid where no cointegration has occurred. In order to specifically isolate only the cointegrates, a second conjugation to another *Agrobacterium* strain without a Ti plasmid *must* be performed. This transfer is mediated by functions encoded by the Ti plasmid itself. Transfer of the intermediate cloning vector into the second *Agrobacterium* strain is effected only in the form of a cointegrate with the Ti plasmid.
e) In order to obtain the final modified Ti plasmid with the desired replacement a second cross-over event is effected (Leemans *et al., J. Mol. Appl. Genet.* 1 (1981), 149-164).

The only other known method is essentially the same as outlined above, except that for step (d) another plasmid which is not compatible with the intermediate cloning vector is introduced into *Agrobacterium;* in this case, the cointegration (single cross-over event) can be selected for since all the intermediate cloning vectors which remain as independent replicons will be lost (Matzke *et al., J. Mol. Appl. Genet.* 1 (1981), 39-49).

We describe a novel and much simplified method for the introduction of intermediate cloning vectors into acceptor Ti plasmids of *Agrobacterium.* Briefly, this method is based on the finding that helper plasmids of *E. coli* allow transfer of many of the commonly used cloning plasmids (such as pBR322) *directly* to *Agrobacterium.* Since none of these plasmids can replicate in *Agrobacterium* only those which can cointegrate with the acceptor Ti plasmid will be retained. In addition, we use this cointegrate in *Agrobacterium* as a vector composition directly for infection of plant cells. In this manner we have eliminated steps (d) and (e) described above which greatly increases the possibilities for using the acceptor Ti plasmids as vectors for DNA transfer to plant cell genomes by both decreasing the time required for constructing modified hybrid Ti plasmids and increasing the flexibility of the possible constructions.

Thus, as outlined in Figure 5, the introduction of the intermediate cloning vector into the acceptor Ti plasmid is accomplished in two steps. First, conjugation of *E. coli* strain (1) carrying the intermediate cloning vector to another *E. coli* strain (2) carrying two plasmids which will help to mobilize the intermediate cloning vector to *Agrobacterium.* Typical and preferred examples of these helper plasmids are R64*drd*11 containing *mob* functions and pGJ28 containing *tra* functions (Finnegan *et al., Mol. Gen. Genet.* 185 (1982), 344-351). A *bom* site (Warren *et al., Nature* 274 (1978), 259-261) on the cloning vehicle of the intermediate cloning vector is recognized by the functions encoded by the other two plasmids to allow transfer to occur. All plasmids should preferably contain antibiotic resistance markers to detect their presence. Secondly, the *E. coli* strain obtained, i.e. the mobilizing strain (3) carrying all three plasmids is conjugated to *Agrobacterium* containing an acceptor Ti plasmid with a region of homology with the intermediate cloning vector. A single cross-over event between the intermediate cloning vector and the acceptor Ti plasmid is detectable by selection for the antiobiotic resistance marker of the intermediate cloning vector.

Figure 6 schematically illustrates the DNA molecules used to construct the acceptor Ti plasmid shown in Figure 1. We call this acceptor Ti plasmid (A) to distinguish it from the other acceptor Ti plasmid (B) (Fig. 8). The construction requires a double cross-over event between a Ti plasmid and another plasmid carrying the border sequences (1) and (2) in a cloning vehicle (3). As illustrated in the Figure, the cloning vehicle sequence (3) lies *between* border sequence (1) on the left and (2) on the right; in order to show the correct polarity of the DNA strands, this can be drawn on a circle. However, to show the regions of homology used in the double cross-over event, this circle has been broken as indicated. This is an important subtlety to realize and it is also used in the construction of acceptor Ti plasmid (B) in Figure 8, i.e., if border sequences (1) and (2) were simply inserted *within* cloning vehicle sequence (3), a double cross-over event would produce a Ti plasmid with a deleted T-region but *without* the cloning vehicle sequence between the border sequences (1) and (2). As also shown here in Figure 6, the double cross-over event also produces a circular DNA molecule which contains the original T-region between border sequences (1) and (2); as this is not a replicon, it will be lost. Genetic selection for these events can be achieved by, for example, selecting for the loss of an antibiotic resistance marker contained within the T-region of the Ti plasmid and selecting for an antibiotic resistance marker contained within the cloning vehicle sequence (3).

Figure 7 schematically illustrates the construction of an intermediate cloning vector of Figs. 2 and 3. A gene of interest (5) and a selectable marker gene (6), each bracketed by a restriction endonuclease site R1 or R2, respectively, are inserted into a cloning vehicle sequence (3') which contains unique restriction sites for enzymes R1 and R2 by digestion and ligation of all molecules. The recombinant DNA molecule obtained is ued to transform *E. coli* host cells and transformants are selected for the antibiotic resistance marker (Ab^{R1}) of the cloning vehicle sequence (3').

Figure 8 schematically illustrates the DNA molecules used to construct another embodiment of the invention, i.e. acceptor Ti plasmid (B). Here a double cross-over event occurs between a Ti plasmid and a plasmid containing the cloning vehicle sequence (3) *between* the DNA sequences (9) and (10), which are located just outside the border sequences (1) and (2); in order to illustrate the homologous regions used in the cross-over event the small plasmid has been broken (as in Fig. 6). The product of the double cross-over event is acceptor Ti plasmid (B) and another circular DNA molecule which is lost containing the T-region from the original Ti plasmid plus the DNA sequences (2), (10), (9), and (1). Genetic selection can be achieved as described for Figure 6.

Figure 9 schematically illustrates an intermediate cloning vector to be used in combination with the acceptor Ti plasmid B of Figure 8. Here, the gene of interest (5) is inserted between the border sequences (1) and (2) which are contained in a cloning vehicle sequence (3').

Figure 10 schematically illustrates how a single cross-over event introduces the intermediate cloning vector of Fig. 9 into the acceptor Ti plasmid (B). In this case, selection for the antibiotic resistance marker of the cloning vehicle sequence (3') of the intermediate cloning vector ensures that a hybrid Ti plasmid can be found which is the result of a cointegration between the two plasmids. A hybrid Ti plasmid is produced with the gene of interest contained within the border sequences (1) and (2). The hybrid plasmid thus constructed does not contain in its T-region a sequence which is a direct repeat (as for example the sequences (3) and (3') in hybrid Ti plasmid of Fig. 4, avoiding possible problems of instability of the hybrid vector or of the transformed DNA in the plant cell genome as a result of intramolecular recombination.

Unpublished results from our laboratory also indicate that for construction of the intermediate vector in Fig. 9, it is not essential to have both border sequences 1 and 2; it is sufficient but essential to have at least the right border sequence (2) (see Fig. 1 and Fig. 9), in order to obtain integration of the desired DNA sequence into the plant genome.

Knowledge of the restriction endonuclease maps of the Ti plasmids of *Agrobacterium,* e.g. nopaline or octopine Ti plasmids (Depicker *et al.*, *Plasmid* 3 (1980), 193-211; De Vos *et al., Plasmid* 6 (1981), 249-253), plus the knowledge of the restriction fragments which contain the T-DNA border regions (Zambryski *et al., J. Mol. Appl. Genet.* 1 (1982), 361-370; De Beuckeloer *et al., Mol. Gen. Genet.* 183 (1981), 283-288) enables now any investigator to construct acceptor Ti plasmids according to the process of this invention. The only additional skill required is the ability to perform conventional recombinant DNA techniques and basic bacterial genetic manipulations. The present invention is unique in that it specifically proposes the described acceptor Ti plasmids which have been shown to be effective for the construction of hybrid Ti plasmid vectors; furthermore these acceptor Ti plasmids are designed to form part of a process to introduce genes into the plant cell genome.

In order to further illustrate the disclosed acceptor Ti plasmids, intermediate cloning vectors, hybrid Ti plasmid vectors and vector compositions, and to demonstrate the effectiveness of the vector compositions in providing transformed plant cells and plants showing expression of the foreign gene(s) integrated into the plant cell genome, the following examples are provided.

### Example 1

### Construction of acceptor Ti plasmid pGV3850 (type A)

Starting strains and plasmids:
*Agrobacterium tumefaciens* (rifampicin-resistant strain C58C1, and chloramphenicol-erythromycin-resistant strain C58C1 derived from wild-type *Agrobacterium)*
Ti plasmid=pGV3839
Plasmid of Fig. 11=pAcgB

The Ti plasmid pGV3839 is constructed from a nopaline plasmid pTiC58tra^{c} (pGV3100; Holsters *et al., Plasmid* 3 (1980), 212-230). It contains a deletion substitution mutation near the centre of the T-region; the *Sma*l fragment 24 internal to *Hin*dlll fragment 19 (Depicker *et al., Plasmid* 3 (1980), 193-211) has been substituted by a *Hin*dll fragment of pKC7 (Rao *et al., Gene* 7 (1979), 79-82) which contains the *apt* (acetylphosphotransferase) gene of Tn*5*. This gene codes for resistance to the aminoglycosides neomycin and kanamycin. Figure 12 gives a restriction map of the T-region of pGV3839.

The plasmid pAcgB is an insert of AcgB in pBR322 which contains only the borders of the T-DNA (see Fig. 11). The borders are defined as the ends of the T-DNA and these regions play a role in the stable integration of the T-DNA into the plant cell genome. The origin and analysis of this clone has been described in detail (Zambryski *et al., Science* 209 (1980), 1385-1391). This clone was obtained by re-isolating portions of the T-DNA from transformed tobacco DNA. pAcgB contains the junction of two T-DNA copies which are arranged in tandem so that it contains the left and right borders of the T-DNA. In addition, pAcgB contains the nopaline synthase gene since this genetic information maps very close to the right T-DNA border. The plasmid pAcgB is used for the construction of an "A-like" acceptor Ti plasmid, pGV3850. Figure 6 outlines the structures involved and Figure 13 gives more precisely the regions of DNA involved in the double cross-over events leading to pGV3850.

The described plasmid pAcgB carries a ColE1-specific *bom* site in the pBR322 portion and can be mobilized from *E. coli* to *Agrobacterium* by using the helper plasmids R64*drd*11 and pGJ28. The plasmids R64*drd*11 and pGJ28 contained in *E. coli* are introduced into the *E. coli* strain carrying pAcgB by conjugation. Transconjugants are selected as ampicillin-resistant (from the pBR322 sequences of pAcgB), streptomycin-resistant (from R64*drd*11) and kanamycin-resistant (from pGJ28) colonies.

The *E. coli* strain carrying all three plasmids is conjugated to *Agrobacterium* strain C58C1 which is rifampicin-resistant and which contains the Ti plasmid pGV3839. The ampicillin-resistance of pBR322 is used to select for the first single cross-over event with the nopaline Ti plasmid. The only way that the ampicillin resistance can be stabilized in *Agrobacterium* is a cross-over event upon homologous recombination with pGV3839 through one of the homology regions near the T-region borders. By a second cross-over event through the other homology region, the central portion of the T-region of pGV3839 including the *apt* gene (kanamycin resistance) is replaced by the pBR322 sequences of the clone pAcgB. Second recombinants are thus ampicillin-resistant and kanamycin-sensitive. To increase the probability of isolating a second recombinant, the rifampicin-resistant *Agrobacterium* carrying the first recombinant (pAgcB::pGV3839) is conjugated with a second chloroamphenicol/erythromycin-resistant *Agrobacterium* strain without a Ti plasmid. In this manner, a chloramphenicol/erythromycin-resistant *Agrobacterium* pGV3850 can be obtained which is ampicillin-resistant and kanamycin-sensitive at a frequency of approximately one in 600 colonies.

Of course, there are other Ti plasmids which can be utilized to construct pGV3850-type acceptor Ti plasmids. Any Ti plasmid carrying a selectable marker gene near the centre of the T-region may be used as a recipient. Furthermore, a pAcgB-like plasmid may be constructed by inserting the T-region border fragments into pBR322 in such a way that the pBR322 sequences lie inbetween the left border fragment and the right border fragment in the orientation left border fragment-pBR322-right border fragment. For example, the left and right border fragments of the nopaline Ti plasmid are *Hin*dlll fragment 10 and 23, respectively (Depicker *et al., Plasmid* 3 (1980), 193-211).

A single cross-over event will introduce an intermediate cloning vector containing any gene of interest which is inserted in pBR322 (or its derivatives) into the modified T-DNA region of pGV3850. The only requirement is that the DNA to be introduced contains an additional resistance marker gene to those already found in pBR322 to use as a means to select for the transfer of the intermediate cloning vector from *E. coli* to *Agrobacterium.* This resistance marker can be contained either within the pBR sequences (such as Cm^{R} for pBR325 or Km^{R} for pKC7) or within the DNA which is to be tested in the plant cell. In addition, in the acceptor Ti plasmid pGV3850 the Ap^{R} gene pBR322 can be replaced by another resistance marker gene such as KmR; in this way even pBR322-containing intermediate cloning vectors which are Ap^{R} can be directly mobilized to this pGV3850-like acceptor Ti plasmid.

An added advantage of the pGV3850-type acceptor Ti plasmid is that it does not produce tumors in transformed plant cells. Cells which have been "transformed" by pGV3850 can easily be screened from untransformed cells by assaying for the presence of nopaline as the shortened T-DNA region of pGV3850 still contains the gene encoding nopaline synthase. Of course, if the intermediate cloning vector which is cointegrated into the acceptor Ti plasmid pGV3850 contains a marker gene it can also be directly screened or selected for.

Besides insertion of defined intermediate cloning vectors containing a pBR322 sequence by a single cross-over event into the acceptor Ti plasmid pGV3850, this acceptor Ti plasmid can also be used as a recipient for cloned banks of DNA in pBR322 or its derivatives in a "shotgun"-type experiment. The total population of hybrid plasmid vectors in *Agrobacterium* can be used to infect plant cells and is subsequently screened for expression of any selectable gene(s) of interest. For example, one can easily select for genes encoding amino acid synthesis by applying the total bank to plant cells which are deficient in the chosen amino acid.

The acceptor Ti plasmid pGV3850 has two phenotypic characteristics: (i) the inability to produce tumors, and (ii) the ability to synthesize nopaline if T-DNA transfer occurs into the plant cell genome. Thus, several experiments are performed to check for these characteristics in various plant tissues infected with *Agrobacterium* containing pGV3850.
a) Tests with potato and carrot discs. Inoculation of potato and carrot slices with the acceptor Ti plasmid pGV3850 results in the production of a small amount of callous tissue. This tissue is tested for the presence of nopaline and is found to be positive. It is interesting that this mutant is able to produce small callous tissue; however, it can only be obtained if the discs are grown in media containing low concentrations of both auxins and cytokinins.
b) Inoculation of whole plants with acceptor Ti plasmid pGV3850. Tobacco and petunia plantlets growing on sterile agar media (without hormones) are inoculated with pGV3850. A small amount of tissue growth can only be observed after several months (normally "wild-type" tumors are detected after two weeks). This tissue does not grow on hormone-free media but can be propagated further as sterile tissue culture on media containing both auxin and cytokinin. This tissue is also shown to be nopaline-positive.
c) Furthermore, since pGV3850 "transformed" cells are not tumor-like, these cells are capable of regenerating into normal plants which still contain in their genome the transferred DNA segment. Normal plants will be obtained by culturing the transformed cells on conventional regeneration media (see also Example 5).

To prove the usefulness of pGV3850 as an acceptor plasmid the following experiment is performed. An intermediate cloning vector containing oncogenic functions of the octopine T-DNA in pBR325 is recombined into *Agrobacterium* harboring pGV3850. The resulting hybrid Ti plasmid in *Agrobacterium* obtained by a single cross-over event is inoculated onto wounded tobacco plants. Tumor tissue develops after about two weeks. This is evidence that the tumor-inducing DNA is reintroduced into pGV3850 and is expressed properly in transformed plant cells.

### Example 2

### Construction of intermediate cloning vectors pGV700 and pGV750

An overview of the constructions is represented schematically in Figure 14. *Hin*dIII fragment 1, representing the right part of TL-DNA of the octopine Ti plasmid B6S3, and present in pGV0201 (De Vos *et al., Plasmid* 6 (1981), 249-253), is inserted first in the *Hin*dlll site of the broad-host range vector pGV1122 (Leemans *et al., Gene* 19 (1982), (361-364). The recombinant plasmid pGV0201 contains the *Hin*dIII fragment 1 inserted in the unique *Hin*dlll site of the multicopy vector pBR322 (Bolivar *et al., Gene* 2 (1977), 95-113). pGV0201 and pGV1122 DNA is prepared as described by Betlach *et al., Fed. Proc.* 35 (1976), 2037-2043. Two µg of pGV0201 DNA are totally digested with 2 units of *Hin*dlll (all restriction enzymes are purchased from Boehringer Mannheim) for 1 hour at 37°C in a final volume of 20 µl. The incubation buffer is described by O'Farrell *et al., Mol. Gen. Genet* 179 (1980), 421-435. Two µg of pGV1122 DNA are totally digested with *Hin*dlll under the same conditions.

One tenth µg of *Hin*dlll digested pGV0201 is ligated to 0.05 µg of *Hin*dIII-digested pGV1122 with 0.02 units of T4 ligase (Boehringer Mannheim) in a final volume of 20 µl. Incubation buffer and conditions are as recommended by the manufacturer (Brochure "T4 ligase", Boehringer Mannheim, August 1980, #10.M.880.486). Transformation of the ligation mixture into competent *E. coli* K514 *hsr⁻ hsm⁺* cells (Colson *et al., Genetics* 52 (1965), 1043-1050) is carried out as described by Dagert and Ehrlich, *Gene* 6 (1980), 23-28. Cells are plated on LB medium (Miller, *Experiments in Molecular Genetics* (1972), Cold Spring Harbor Laboratory, New York) supplemented with streptomycin (20 µg/ml) and spectinomycin (50 µg/ml). Transformants containing recombinant plasmids are screened for tetracycline sensitivity (10 µg/ml), due to the insertional inactivation of the gene coding for tetracycline resistance (Leemans *et al., Gene* 19 (1982), 361-364). Streptomycin- and spectinomycin-resistant and tetracycline-sensitive clones are physically characterized. Microscale DNA preparations are performed according to Klein *et al. (Plasmid* 3 (1980), 88-91). The orientation of the *Hin*dlll fragment 1 in the *Hin*dlll site of pGV1122 is determined by *Sa/*I digestion. Digestion (conditions according to O'Farrel *et al., Mol. Gen. Genet.* 179 (1980), 421-435) of recombinant plasmids gives 2 fragments after agarose gel electrophoresis. In the α-orientation there are fragments of 0.77 kb and 22.76 kb, whereas in the β-orientation there are fragments of 10.33 kb and 13.20 kb. A recombinant plasmid with the α-orientation is used in subsequent cloning and called pGV1168.

A *Bg*/II-*Sa*/I fragment containing the left part of the TL-DNA (including the left border sequence) is introduced in pGV1168, cut with *Bg*/II-*Sa*/I. This fragment is obtained from the recombinant plasmid pGV0153 (De Vos *et al., Plasmid* 6 (1981), 249-253), containing *Bam*HI fragment 8, from the T-region of pTiB6S3, inserted in the vector pBR322. pGV0153 and pGV1168 DNA is prepared according to Betlach *et al. (Fed. Proc.* 35 (1976), 2037-2043). Ten µg of pGV0153 DNA are completely digested with 10 units of *Bg/*II and 10 units of *Sa*/I for 1 hour at 37°C in final volume of 100 µl. The digestion mixture is loaded on a preparative 0.8% agarose gel. The 2.14 kb *Blg*II-*Sa*/I fragment is recovered from this gel by electroelution as described by Allington *et al., Anal. Biochim.* 85 (1978), 188-196. Two µg of pGV1168 DNA are totally digested by 2 units of *Bg*/II and 2 units of *Sa*/I. One tenth µg of *Bg*/II-*Sa*/I fragment DNA is ligated to 0.02 µg of *Bg*/III-*Sa*/I-digested pGV1168 with 0.02 units of T4 DNA ligase in a final volume of 20 µl. The ligation mixture is transformed into competent *E. coli* K514 *hsr⁻ hsm⁺* cells (Dagert and Erhlich, *Gene* 6 (1980) 23-28). Cells are plated on LB medium (Miller, *Experiments in Molecular Genetics* (1972), Cold Spring Harbor Laboratory, New York), supplemented with streptomycin (20 µg/ml) and spectinomycin (50 µg/ml).

Microscale DNA preparations (Klein *et al., Plasmid* 3 (1980), 88-91) are performed from streptomycin- and spectinomycin-resistant transformants. Recombinant plasmids in which 2.14-kb-*Bg*/II-*Sa*/I fragment is inserted in *Bg*/II-*Sa*/I-digested PGV1168 are identified by *Bg*/II-*Sa*/I digestion, yielding 2 fragments of 2.14 kb and 21.82 kb. A plasmid with a digest pattern corresponding to these molecular weights (2.14 kb and 21.82 kb) is used for further cloning and called pGV1171. A 12.65-kb-fragment from pGV1171 contains the right and left TL-DNA border sequences (De Beuckeleer *et al.,* in *Proceedings /Vth International Conference on Plant Pathogenic Bacteria,* M Ridé (ed.) (1978), I.N.R.A., Angers, 115-126), as well as genes which permit oncogenic proliferation (Leemans *et al., EMBO J.* (1982), 147-152). This *Hin*dlll fragment is inserted into the plasmid pBR325 (Bolivar, *Gene* 4 (1978), 121-136). pGV1171 and pBR325 are prepared according to Betlach *et al., Fed. Proc.* 35 (1976), 2037-2043). Two µg of each DNA are totally digested with 2 units of *Hin*dlll for 1 hour at 37°C (incubation buffer is described by O-Farrell *et al., Mol. Gen. Genet.* 179 (1980), 421-435). One tenth µg of *Hin*dIII-digested pGV1171 is ligated to 0.05 µg of pBR325, linearized with *Hin*dlll, with 0.02 units T4 DNA ligase. Transformation of the ligation mixture in competent *E. coli* K514 *hsr⁻ hsm⁺* is carried out as described by Dagert and Ehrlich, *Gene* 6 (1980), 23-28. Cells are plated on LB medium (Miller, *Experiments in Molecular Genetics* (1972), Cold Spring Harbor Laboratory, New York), supplemented with carbenicillin (100 µg/ml). Carbenicillin-resistant clones are screened for sensitivity to tetracyline (10 µg/ml), due to insertional inactivation of the gene coding for tetracycline resistance (Bolivar, *Gene* 4 (1978) 121-136). Carbenicillin-resistant and tetracycline-sensitive clones are physically characterized by restriction enzyme digestion of DNA prepapred from these clones by the microscale technique (Klein *et al., Plasmid* 3 (1980), 88-91). *Bam*HI digestion gives 4 DNA fragments: in the α-orientation fragments of 0.98 kb, 4.71 kb, 5.98 kb, and 7.02 kb are found whereas the β-orientation givs fragments of 0.98 kb, 4.71 kb, 1.71 kb, and 11.20 kb. A recombinant plasmid corresponding to the α-orientation is obtained, called pGV700, and used for further experiments.

pGV750 is derived from pGV700 by inserting a 2.81 kb *Bam*HI*-Hpa*I fragment coding for kanamycin resistance, for a 3.49-kb-*Bg*/II-*Sma*I fragment, encoding functions essential for oncogenicity, internal to the TL-region inserted in pGV700. The *Bam*HI*-Hpa*I fragment encoding kanamycin resistance is obtained from λ::Tn*5* (Berg *et al., Proc. Natl. Acad. Sci. USA* 72 (1975), 3268-3632). Preparation of λ::Tn*5* is as described (Miller, *Experiments in Molecular Genetics* (1972), Cold Spring Harbor Laboratory, New York). pGV700 DNA is prepared according to Betlach *et al.(Fed. Proc.* 35 (1976), 2037-2043). Two µg of pGV700 DNA are totally digested with 2 units of *Bg*/I and 2 units *Sma*l. Two µg of λ::Tn*5* DNA are totally digested with 2 units of *Bam*HI and 2 units *Hpa*l. One µg of *Bam*HI*-Hpa*I digested λ::Tn*5* is ligated to 0.2 µg *Bg*/II-*Sma*I-digested (pGV700 with 0.5 units of T4 DNA ligase in a final volume of 10 µl (conditions are as recommended by the manufacturer). The ligation mixture is transformed in competent *E. coli* K514 *hsr⁻ hsr⁺* cells (Dagert and Erhlich, *Gene* 6 (1980) (23-28). Cells are plated on LB medium (Miller, *Experiments in Molecular Genetics* (1972), Cold Spring Harbor Laboratory, New York), supplemented with carbenicillin (100 µg/ml) and kanamycin (25 µg/ml). Cb^{R} and Km^{R} clones are physically characterized by restriction enzyme analysis of DNA prepared according to the microscale technique (Klein *et al., Plasmid* 3 (1980), 88-91). *Bg*/II/*Bam*HI double digests of this DNA gives 3 fragments of 3.94 kb, 5.89 kb, and 8.09 kb, whereas *Hin*dlll digests yields 3 fragments of 2.68 kb, 5.99 kb, and 9.25 kb. A plasmid showing these digestion patterns is called pGV750 and is illustrated schematically in Figure 17.

pGV700 and pGV750 are two different intermediate cloning vectors which contain the left and right border sequences of TL-DNA of the octopine Ti plasmid pTiB6S3; in addition, the internal T-region is deleted to different extents in each of the two plasmids. pGV700 contains a shortened T-region with genetic information for octopine synthase (transcript 3), and 3 other products, 4, 6a, and 6b (see Willmitzer *et al., EMBO J.* 1 (1982), 139-146, for a description of T-region products). The combination of these three products (4, 6a, 6b) will cause shoot formation in transformed plants. pGV750 contains even less of the T-region, i.e. only the octophine synthase gene. The information for products 4, 6a, and 6b has been substituted by the antibiotic resistance marker gene encoding kanamycin (neomycin) resistance.

pGV700 and pGV750 are examples of intermediate cloning vectors which can be used with acceptor Ti plasmids of the B-type (see Fig. 8 and Example 3 below); these vectors are partially analogous to the one shown in Fig. 9 except that they do not contain a gene of interest. A gene of interest can be easily inserted into these vectors as they contain single restriction endonuclease sites for cloning within their modified T-regions (see Figs. 14 and 15).

### Example 3

### Construction of acceptor Ti plasmid pGV2260 (type B)

Starting strains and plasmids:
*Agrobacterium tumefaciens* rifampicin-resistant strain C58C1 and erythromycin-chloroamphenicol-resistant strain C58C1, derived from wild-type *Agrobacterium)*
   Ti plasmid=pGV2217
   Intermediate vector (Fig. 16)=pGV745

The construction of the Ti plasmid pGV2217 has been described in detail (Leemans *et al., EMBO J.* 1 (1982), 147-152). It contains a deletion substitution mutation of the entire TL-region of the octopine Ti plasmid: the *Bam*HI fragments 8, 30b, 28, 17a and the left 3.76 kb *Bam*Hi-*Eco*RI fragment of the *Bam*HI fragment 2 (De Vos *et al., Plasmid* 6 (1981), 249-253) have been substituted by an *Eco*RI*-Bam*HI fragment of pKC7 (Rao & Rogers, *Gene* 7 (1979), 79-82) which contains the *apt* (acetyl phosphotransferase) gene of Tn*5*. This gene codes for resistance to the aminoglycosides, neomycin and kanamycin.

The construction of the intermediate vector pGV745 is represented schematically in Fig. 16 and is described as follows. The recombinant plasmid pGV713 was derived from the octopine Ti plasmid subclone pGV0219 (De Vos *et al., Plasmid* 6 (1981), 249-253), containing *Hin*dlll fragments 14, 18c, 22e and 38c in α-orientation. pGV0219 DNA was digested to completion with *Bam*HI and subsequently ligated under conditions which favour self ligation of the plasmid (final concentration of DNA in ligation mixture <1 µg DNA/ml). Transformants were selected for ampicillin resistance, and physically characterized by restriction enzyme digestion. A clone, which no longer contains the 6.5 kb *Bam*HI fragment present in pGV0219, was isolated and called pGV713. This clone pGV713 was used in subsequent cloning (see below). The recombinant plasmid pGV738 was derived from pGV0120 (De VOs *et al., Plasmid* 6 (1981), 249-253), containing *Bam*HI fragment 2. pGV0120 DNA was digested with *Eco*RI and self-ligated (as above for the construction of pGV713). Transformants were selected for ampicillin resistance and analyzed by restriction enzyme digestion. A clone in which *Eco*RI fragments 20, 12, and a 2.95 kb *Eco*RI fragment containing part of *Eco*RI fragment 19a and part of pBR322 were deleted, was used in further cloning and called pGV738. This plasmid still contains a 5.65 kb *Eco*RI*-Bam*HI fragment from the right part of *Bam*HI fragment 2 (De Vos *et al., Plasmid* 6 (1981), 249-253).

Next, pGV713 DNA was digested with *Hin*dlll and *Bam*HI, and the digest was applied onto a preparative agarose gel. After electrophoresis the 2.30 kb *Hin*dIII-*Bam*HI fragment, contained within pGV713, was purified by electroelution (as described by Allington *et al., Anal. Biochem.* 85 (1975), 188-196). This fragment was ligated to pGV738, digested to completion with *Hin*dIII and *Bam*HI. After transformation, ampicillin-resistant clones, were physically characterized by restriction enzyme digestion. For example, *Eco*RI-*Bam*HI digestion should give 2 fragments of respectively 3.98 kb (= vector part) and 7.95 kb (= insert part). A recombinant plasmid with these characteristics was called pGV745 and used as intermediate vector for the construction of the acceptor Ti plasmid pGV2260.

The plasmid pGV745 contains a ColE1-specific *bom* site in the pBR322 portion and can be mobilized from *E. coli* to *Agrobacterium* by using the helper plasmids R64*drd*11 and pGJ28, as described in example 1 (construction of the acceptor Ti plasmid pGV3850).

pGV745 was mobilized to *Agrobacterium* strain C58C1 which is rifampicin-resistant and contains the Ti plasmid pGV2217. The first cross-over event was selected by using the ampicillin resistance of pBR322 in the same way as described in Example 1 (the construction of the acceptor Ti plasmid pGV3850). By a second cross-over event the deletion substitution mutation present in pGV2217 is replaced by the pBR322 sequences of the plasmid pGV745. Second recombinants were picked up by directly screening the ampicillin-resistant transconjugants, which resulted from cointegration of pGV745 with pGV2217, for the loss of kanamycin resistance. In this way, a rifampicin *Agrobacterium* strain C58C1, containing pGV2260 (ampicillin-resistant, kanamycin-sensitive), was obtained.

This Ti plasmid pGV2260 will be used as an acceptor plasmid (type B) for intermediate cloning vectors of the pGV700- or pGV750-type. These are composed of (i) a DNA fragment carrying the ampicillin resistance gene, the origin of replication and the *bom* site of pBR322; (ii) a DNA fragment containing the left and right border sequences of the TL-DNA, and an additional resistance marker to those already present on pBR322, which will enable the genetic selection for the transfer of the intermediate cloning vector from *E. coli* to *Agrobacterium* as well as for its cointegration in the acceptor Ti plasmid pGV2260.

For example, we have been able to show that *Agrobacterium* carrying a cointegrate between pGV2260 and pGV700 is capable of transferring the expected DNA sequences (those contained between the T-DNA borders) to plant cell genome. The transformed plant cells exhibit the expected phenotype i.e. tumors which produce shoots, given that pGV700 contains the genetic information for three products (4, 6a, 6b; see Willmitzer *et al., EMBO J.* 1 (1982), 139-146). In this manner we have shown that an acceptor Ti plasmid of the B-Type is capable of transferring DNA to plant cells when used as a cointegrate with an intermediate cloning vector of the type illustrated in Fig. 9 and given in example 2.

### Example 4

### Construction of an intermediate cloning vector containing a gene to be expressed in plants

Until the present invention, insertion of whole genes into more or less random positions within the T-region of Ti plasmids has not resulted in expression of the foreign sequence following transfer to the plant genome. According to the process of this invention, the coding region of (any) foreign gene(s) of interest is linked to transcriptional initiation and termination signals which are known to be functional in the plant cell. The usefulness of this approach is demonstrated according to the present invention by experiments involving the DNA sequences encoding the nopaline synthase gene. The entire sequence of this gene and the exact start and stop of transcription are known (Depicker *et al., J. Mol. Appl. Genet.* 1 (1982), 561-574). According to the present invention, the protein-coding region of any foreign gene can be inserted adjacent to the *nos* promoter. As an example of a foreign gene sequence, the coding region of the octopine synthase gene (De Greve *et al., J. Mol. Appl. Genet.* 1 (1982), 499-512) is inserted adjacent to the *nos* promoter. This construction is mobilized into an acceptor Ti plasmid and used to infect plants. The resulting tumor tissue is assayed for the presence of octopine, and is found to be positive.

The construction of the intermediate cloning vector containing the chimeric nopaline promoter: octopine synthase structural gene is shown and described in Figures 18 to 20.

Briefly, the restriction fragment *Hin*dlll-23 containing the *nos* gene is engineered *in vitro* to remove most of the *nos* coding sequence, and retain the *nos* promoter adjacent to the restriction endonuclease site *Bam*HI (Fig. 18). Ten µg of pGV0422 (a pBR322 derivative carrying the *Hin*dIII-23 fragment which contains the complete *nos* gene; Depicker *et al., Plasmid* (1980), 193-211) are digested with *Sau*3A and the 350 bp fragment carrying the *nos* promoter is isolated from a preparative 5% polyacrylamide gel. The promoter fragment is ligated to *Bg*/II-cut pKC7 (Rao *et al*., *Gene* 7 (1979), 79-82) previously treated with bacterial alkaline phosphatase (BAP) to remove 5'-terminal phosphate groups. Twenty µg of the resulting plasmid (pLGV13) are digested with *Bg*/II and treated with 7 units of the *Ba*/31 exonuclease (Biolabs, New England) for 4-10 minutes in 400 µl of 12 mM MgCl₂,12 mM CaCl₂, 0.6 M NaCl, 1 mM EDTA, and 20 mM Tris-HCl, pH 8.0, at 30°C. During this time approximately 20-50 bp of DNA are removed. The *Ba*/31-treated molecules are digested with *Bam*HI, and incubated with the Klenow fragment of DNA polymerase and all four deoxynucleoside triphosphates (at 10 mM each) to fill in the ends. Plasmids are screened for a regenerated *Bam*HI site derived from the ligation of a filled-in *Bam*HI end and the end of the *Ba/*31 deletion. The sizes of the *Bam*Hl-*Sac*ll fragment of several candidates are estimated in a 6% urea-polyacrylamide gel, and the nucleotide sequences of the candidates with sizes ranging between 200-280 nucleotides are determined. The clone pLGV81 containing the *Sac*II*-Bam*HI fragment of 203 bp carrying the promoter is used to substitute the *Sac*II*-Bam*HI fragment in the *nos* gene in pGV0422; the final promoter vector is called pLGV2381. All the recombinant plasmids are selected by transformation of the *E. coli* strain HB101.

The plasmid vector containing the engineered *nos* promoter is digested with *Bam*HI and the coding sequence for *ocs* which is contained on a *Bam*HI fragment is inserted into this site. The *ocs* coding sequence is also engineered *in vitro* to be bracketed by the *Bam*HI restriction endonuclease site as described in Fig. 19. Ten µg of *Bam*HI fragment 17a of the octopine Ti plasmid B6S3 (De Vos et *al., Plasmid* 6 (1981), 249-253) are digested with *Bam*HI and *Sma*I*,* the fragment containing the *ocs*-coding sequence isolated from a 1% agarose gel, and ligated to the large *Bam*HI*-Pvu*II fragment of pBR322; 20 vg of the resulting plasmid, pAGV828, is digested with *Bam*HI, treated with the exonuclease *Ba/*31 as described in Figure 18, subsequently digested with *Hin*dIII, and the ends are filled-in and self-ligated. The sizes of the *Ba*/31 deletions are estimated in a 6% polyacrylamide gel. The nucleotide sequences of several candidates are determined, and a candidate having only 7 bp remaining of the 5'-untranslated leader sequence is chosen for further work )pOCSΔ). In order to bracket the *ocs* sequence with *Bam*HI sites, the *Cla*I*-Rsa*I fragment is filled-in and subcloned into the *Ba/*I site of pLC236 (Remaut *et al*., *Gene* 15 (1981), 81-93). The resulting plasmid pAGV40 is digested with *Bam*HI*,* the fragment carrying the *ocs* sequence isolated by electroelution from a preparative 1% agarose gel, and ligated to pLGV2381 previously cut with *Bam*HI, and treated with BAP (bacterial alkaline phosphatase). The insertion of the *ocs* sequence in pLGV2381 is obtained in both orientations (pNO-1 and pNO-2).

The nucleotide sequences showing the exact junction point in the *nos:ocs* fusion are shown in Fig. 20.

Further, the plasmid vector containing the engineered *nos* promoter is used to insert DNA from the plasmid R67 which encodes the enzyme dihydrofolate reductase. The coding sequence containing the dihydrofolate reductase gene is contained on a *Bma*HI fragment as described (O'Hare *et al., Proc. Natl. Acad. Sci. USA* 78 (1981), 1527-1531), and thus is easily inserted into the *nos* promoter vector containing the *Bam*HI site adjacent to the promoter region as described above. This gene is an example of a selectable marker gene (see for example Figs. 2, 3, 4, 5, and 7) since when expressed it provides resistance to the antibiotic methotrexate. When this intermediate cloning vector is mobilized into an *Agrobacterium* containing a wild-type nopaline acceptor Ti plasmid, a single cross-over event occurs and a hybrid Ti plasmid vector is obtained. The vector composition is used to infect plants. The resulting tumor tissue is found to be capable of sustained growth in the presence of 0.5 µg/ml methotrexate.

The construction of the intermediate cloning vectors containing the *ocs* and dihydrofolate reductase coding regions behind the *nos* promoter described above, and their transfer and expression in transformed plant cells following cointegration with the Ti plasmid *of Agrobacterium* provides evidence that foreign genes can be transferred and expressed in plant cells according to the processes of this invention.

### Example 5

### Isolation of plant cells and plants containing the desired gene(s) inserted in their chromosomes

We have obtained plant cells and whole plants transformed with non-oncogenic acceptor Ti plasmid derivatives (e.g. pGV3850) using any of the following three methods:
(1) inoculation *in vivo* of whole plants followed by subsequent culture *in vitro* on media which allow the regeneration of shoots;
(2) coinfection *in vivo* of whole plants in the presence of other *Agrobacteria* strains which directly induce shoots at the wound site;
(3) cocultivation *in vitro* of single plant cell protoplasts.

We will describe each of these methods below.

The first method is based on a modification of methods normally used to obtain infection of whole plant tissues with wild-type *Agrobacterium* strains which results in the production of crown gall tissues. Since pGV3850 is a non tumor-producing (non-oncogenic) *Agrobacterium* derivative, no tumorous growth is observed at the infected site. However, if the infected tissue is removed and propagated in tissue culture, transformed tissues can easily be obtained. After an initial culture period (simply to increase the mass of the tissue) the wound site tissue is grown under conditions which allow shoots to form. Both untransformed and pGV3850-transformed cells will produce shoots. The transformed shoots can easily be distinguished by a simple assay for the presence of nopaline.

We have obtained pGV3850-transformed calli and shoots derived from decapitated tobacco plantlets of *Nicotiana tabacum* Wisconsin 38 using the following protocol (all manipulations are done under sterile conditions in a laminar flow hood).
(1) Use 6-week old tobacco seedlings grown in small jars (10 cm diameter×10 cm height) on solid Murashige & Skoog (MS) medium (Murashige and Skoog, *Physiol. Plant.* 15 (1962) 473-497) containing 0.8% agar.
(2) Remove youngest top leaves with a scalpel and discard.
(3) Inoculate wound surface with a spatula or toothpick containing *Agrobacterium* derived from a fresh plate culture grown under selective conditions (e.g. for the rifampicin-resistant, ampicillin resistant *Agrobacterium* strain containing Ti plasmid pGV3850, YEB medium containing 100 µg/ml rifampicin and 100 µg/ml carbenicillin are used; YEB medium: 5 g/l Bacto beef extract, 1 g/l Bacto yeast extract, 5 g/l peptone, 5 g/l sucrose, 2×10⁻³ M MgSo₄, pH 7.2, and 15 g/l agar). Inoculate at least 8 plantlets for each pGV3850 construction.
(4) Incubate 2 weeks; there should be little or no response at the site of inoculation; sometimes *very* tiny calli appear.
(5) Remove a thin section less than 1 mm thick from the wound surface.

Incubate wound surface on a plate containing Linsmaier & Skoog (LS) agar medium (Linsmaier and Skoog, *Physiol. Plant.* 18 (1965) 100-127) with auxins and cytokinins (1 mg/l NAA, 0.2 mg/l BAP) and 1% sucrose.
(6) After about 6 weeks callus should be large enough, about 5 mm diameter at least, to test a portion for the presence of nopaline. Not all wound calli produce nopaline; approximately one in four plants produce a nopaline-positive wound callus.
(7) Transfer nopaline-positive calli to agar plates containing regeneration medium: LS medium as above +1% sucrose and 1 mg/l BAP cytokinin.
(8) Good-sized shoots (1 cm high) appear after about 4-6 weeks. Transfer the shoots to fresh agar plates containing LS medium +1% sucrose *without* hormones to allow further growth and root formation.
(9) Let shoots grow 1 or 2 weeks such that a portion (one or two small leaves) can be removed to test for the presence of nopaline.
(10) Transfer nopaline-positive shoots to larger vessels (10 cm jars as above containing MS medium as in (1) to grow further.

N.B. All plant culture media for infected tissues contain 500 µg/ml of the antibiotic cefotaxime (Claforan®, Hoechst) as a selection against *Agrobacterium* containing pGV3850. This drug works well to prevent growth of all *Agrobacteria* (including those which are carbenicillin-resistant).

Another method to obtain transformed and shooting tissues has been recently developed in our laboratory. This method is based on the observation that certain mutant Ti plasmid strains of *Agrobacterium* induce crown gall tumors which produce shoots. Such shoot-inducing *(shi)* mutants map to a particular region of the T-DNA (transferred DNA segment) of the Ti plasmids of *A. tumefaciens* (Leemans *et al., EMBO J.* 1 (1982) 147-152; Joos *et al., Cell* 32 (1983) 1057-1067). Often the induced shoots are composed of completely normal untransformed cells. Thus, we tried to inoculate plants with a mixture of two different *Agrobacteria,* one carrying anoctopine Ti plasmid shooter mutant and the other carrying pGV3850. In this manner, there is a good chance that the octopine shooter mutation can induce shoots which have been transformed with pGV3850. We have inoculated plants with *Agrobacterium* containing Ti plasmid pGV3850 and an octopine shoot inducing Ti plasmid in a 5:1 ratio. In this way we have obtained pGV3850-transformed shoots; these shoots are easily screened by assaying for the presence of nopaline. This method avoids the need for any elaborate tissue culture methods. The nopaline-positive shoots are transferred to media containing simple salts and sugar with any growth-regulating hormones to allow further growth. After the shoots have reached a sufficient size they can easily be transferred to soil for propagation. This coinfection procedure should be particularly useful for transforming plant species which are not readily amenable to tissue culture. Thus, a whole range of agronomically and economically important plants, such as legumes, medicinal plants, and ornamentals will be able to be engineered by *Agrobacterium.*

The third procedure allows the isolation of *Nicotiana tabacum* protoplasts and the selection of hormone-independent T-DNA-transformed cell clones after co-cultivation of the protoplast-derived cells with oncogenic *Agrobacterium* strains. An analogous technique can be used for the selection of transformed cells when other dominant selective markers are used, such as antibiotic resistance genes constructed in such a way as to be expressed in higher plant cells (see Example 3).

In this case, however, the conditions for selection have to be optimized in each case (concentration of the selective agent, time between transformation and selection, concentration of the protoplast-derived cells or cell colonies in the selective medium). If no selection for the transformed cells is possible, e.g. because avirulent T-DNA mutants are used, such as e.g. pGV3850 or pGV2217 (Leemans *et al., EMBO J.* 1 (1982), 147-152), it is possible to cultivate the cells after genetic transformation on auxin- and cytokinin-containing medium (e.g. Murashige and Skoog medium (Murashige and Skoog, *Physiol. Plant 15* (1962), 473-497) with 2 mg/litre NAA (α-naphthalene acetic acid) and 0.3 mg/litre kinetin), and to identify the transformed colonies by their opine content. In this way, after electrophoretic analysis for agropine and mannopine synthesis (method see Leemans *et al., J Mol. Appl. Genet.* 1 (1981), 149-164) about 660 colonies can be found, obtained after infection with pGV2217, a *Nicotiana tabacum* SR1 cell line which synthesizes the TR-encoded opine mannopine (N-(1-mannityl)-glutamine). Numerous shoots are formed after incubation of callus pieces of this cell line on regeneration medium (Murashige and Skoog medium with BAP (6-benzylaminopurine) (1 mg/litre) as the sole plant growth regulator). All 20 shoots analyzed are still able to synthesize mannopine. After transfer onto hormone-free Murashige and Skoog medium, the shoots grow as morphologically normal tobacco plants still containing mannopine.

The protoplast isolation and transformation described here for *N. tabacum* can also be used for *N. plumbaginifolia.*

### 2. Experimental procedures

### 2.1 Shoot culture conditions

*Nicotiana tabacum* shoot cultures are maintained in 250 ml glass jars on hormone-free Murashige and Skoog medium (Murashige and Skoog, *Physiol. Plant* 15 (1962), 473-497) under sterile conditions in a culture room (16 hour day, 1500 lux white fluorescent light ("ACEC LF 58 W/2 4300°K Economy"), 24°C, 70% relative humidity). Five week old shoot cultures are used for protoplast isolation.

### 2.2 Protoplast isolation

Aseptic techniques are used for all steps in protoplast isolation and culture. The protoplasts are isolated by a mixed enzyme procedure. All leaves, except for very young leaves smaller than 2 cm, can be used for protoplast isolation. The leaves are cut in strips, about 2-3 mm wide, with a sharp scalpel knife. Two to three grams of leaf material is incubated 18 hours at 24°C in 50 ml enzyme mixture in the dark without agitation. The enzyme mixture consists of 0.5% cellulase Onozuka R-10 and 0.2% macerozyme Onozuka R-10 in hormone-free K3 medium (Nagy and Maliga, *Z. Pflanzenphysiol.* 78 (1976), 453-455). The mixture is filter-sterilized through a 0.22 µm pore membrane, and can be stored for at least 6 months at -20°C without notable loss of activity.

### 2.3 Protoplast culture

After 18 hours incubation the mixture is agitated gently in order to release the protoplasts. The mixture is subsequently filtered through a 50 µm sieve, and the filtrate is transferred to 10 ml centrifuge tubes. After centrifugation for 6 minutes at 63-80 g in a swinging bucket rotor the protoplasts form a dark green floating band. The liquid underlying the protoplasts, and the debris which forms the pellet, are removed using a capillary tube connected to a peristaltic pump. The protoplasts are pooled in one tube and washed 2 times with culture medium. The culture medium is the K3 medium (Nagy and Maliga, *Z. Pflanzenphysiol.* 78 (1976), 453-455) with NAA (0.1 mg/litre) and kinetin (0.2 mg/litre) as growth regulators. The medium is adjusted to pH 5.6 and sterilized through 0.22 µm filter membrane. After the second wash, the protoplasts are counted using a Thoma hemacytometer (obtained from "Assistant", F.R.G.), and resuspended in culture medium at a final density of 10⁵ protoplasts/ml. The protoplasts are cultured in a volume of 10 ml per 9 cm diameter tissue culture quality petri dish. The dishes are sealed with Parafilm® and incubated for 24 hours in the dark, and thereafter in dim light (500-1000 lux) at 24°C.

### 2.4 Transformation by co-cultivation

The protoplast cultures are infected 5 days after isolation. *Agrobacterium* cultures are grown for 18 hours in liquid LB medium (Miller, *Experiments in Molecular Genetics* (1972), Cold Spring Harbor Laboratory, New York), and resuspended in K3 culture medium at a density of 2×10⁹ cells/ ml. Fifty µl of this suspension is added to the plant protoplast cultures and after sealing with Parafilm®, the cultures are incubated under the same conditions as described under 2.3. After 48 hours the cultures are transferred to 10 ml centrifuge tubes and centrifuged in a swinging bucket rotor at 63-80 g for 6 minutes. The floating band and pellet are pooled and resuspended in 10 ml of K3 medium (Nagy and Maliga, Z. *Pflanzenphysiol.* 78 (1976), 453-455) supplemented with an antibiotic (carbenicillin 1000 µg/ml or cefotaxime 500 µg/ ml).

After two weeks of incubation, the protoplast-derived micro-calli are centrifuged and resuspended in K3 medium (Nagy and Maliga, *Z. Pflanzenphysiol.* 78 (1976), 453-455) with the same growth regulator and antibiotic concentrations as before, but 0.3 M sucrose instead of 0.4 M. The cell density in this medium is adjusted to about 25×10³ micro-calli per ml.

After two weeks of incubation under the same conditions the calli are transferred to K3 medium with the same antibiotic concentrations as before, but with reduced sucrose (0.2 M) and growth regulators (NAA 0.01 mg/litre and kinetin 0.02 mg/ litre).

After two to three weeks of incubation, the putative transformants can be recognized by their light green and compact aspect, and better growth. These colonies are then transferred to hormone-free Linsmaier and Skoog medium (Linsmaier and Skoog, *Physiol. Plant.* 18 (1965), 100-127), solified with 0.6% agar and containing reduced antibiotic concentrations (carbenicillin 500 µg/ml or cefotaxime 250 µg/ml).

Opine tests can be done on the putative transformants which grow on this hormone-free medium when they reach 3-4 mm in diameter. Half of each colony is used for the detection of octopine and nopalin (Aerts *et al., Plant Sci. Lett.* 17 (1979), 43-50) or agropine and mannopine (Leemans *et al., J. Mol. Appl. Genet.* 1 (1981), 149-164). This test allows to confirm the transformed nature of the colonies selected on hormone-free medium. Afterwards, the selected colonies can be cultured on antibiotic-free medium.

### 2.5 Co-cultivation without selection on hormone-free media

When selection for transformed cells is not possible (e.g. because avirulent T-DNA mutants are used) or is not required because a dominant selectable marker such as an antibiotics resistance gene is present in the T-DNA, the treatment of the protoplast-derived cells can be simplified (the hormone reduction steps are no longer necessary). The protoplasts are treated as described previously until the infection step. Forty-eight hours after addition of the bacteria the protoplast-derived cells are centrifuged (6 minutes, 60-80 g), and resuspended in medium AG (Caboche, *Planta* 149 (1980), 7-18) which is able to support cell growth at very low density. The cells are counted using a Fuchs-Rosenthal counting chamber (obtained from "Assistant", F.R.G.), and resuspended at the density required for subsequent work. If the colonies must be manipulated individually for opine tests, plating at low cell density (100 protoplast-derived cells and cell colonies per ml) gives large cell colonies after one month of incubation. If drug selection for the transformed cells is possible, the cells are incubated at a higher density (10³-10⁴/ml), and the selective agent used is added to the medium in a concentration and at a time which has to be optimized for each type of selection.

### 2.6 Regeneration of whole plant from callous tissue

Normal plants are easily obtained from callous tissue (for example either derived from protoplast transformation or from whole plant inoculation (see 2.7). The callous tissue is grown on Murashige and Skoog medium containing 1 mg/ ml BAP; this medium induces shoot formation after 1-2 months. These shoots can be transferred to medium without hormones so that roots form and a complete plant is produced.

### 2.7 Tumor induction on tobacco seedlings

Tobacco seeds (e.g. cultivar Wisconsin 38) are surface sterilized by treatment with: 70% denatured ethanol/H₂O for 2 minutes; followed by 10% commercial bleach and 0.1% sodium dodecyl sulfate (SDS); further rinsed 5 times with sterile H₂O.

The sterile seeds are sown in large (25 mm wide) test tubes containing the salts of Murashige and Skoog medium solidified with 0.7% agar and covered with polycarbonate tops. Then the tubes are incubated in culture room (12,000 lux, 16 hours light/8 hours dark; 70% relative humidity; 24°C). After 4-6 weeks the plants are ready to use. They remain optimal for at least another month.

Plantlets should be at least 3 cm high and have four or more leaves. The plants are then decapitated transversally through the youngest internode with a new sterile scalpel blade; the upper part of the plant removed from the tube, and bacteria from an agar plate culture applied on the wound surface with a flamed microspatula.

Tumors appear after 2 weeks for the wild-type and after a longer time for some of the attenuated mutant strains. This method is used to inoculate tobacco *(Nicotiana tabacum), Nicotiana plumbaginifolia* and *Petunia hybrida.*

### Concluding remarks

The present invention offers for the first time the possibility to transform plants with *Agrobacterium* harboring a hybrid Ti plasmid vector which lacks the oncogenic functions of the T-region of wild-type Ti plasmids. Since the influence of the oncogenic functions of the T-region on the transfer of DNA from the Ti plasmid to plant cells was not known, it is surprising that nevertheless transfer of the modified T-region containing (a) gene(s) of interest to plant cells occurs. There is cointegration and stable maintenance of this transferred DNA in the plant cell genome. Furthermore, expression of chosen gene(s) of interest can be achieved provided the gene(s) either contain-or are constructed to contain-suitable promoter sequences. The concept of effecting a single cross-over event between an intermediate cloning vector containing the chosen gene(s) of interest with an especially designed acceptor Ti plasmid greatly simplifies the construction of any hybrid Ti plasmid vector useful for the transformation of plant cells. The especially designed acceptor Ti plasmids contain the DNA segment of a conventional cloning vehicle such that any gene(s) of interest (which has been inserted into the same or a related cloning vehicle as part of an intermediate cloning vector) can form a cointegrate by a single cross-over event. The two segments of the cloning vehicle(s) provide the necessary regions of homology for recombination.

Microorganisms and intermediate cloning vectors, acceptor Ti plasmids and hybrid plasmid vectors prepared by the processes of this invention are exemplified by cultures deposited in the German Collection of Microorganisms (DSM), Göttingen, on December 21st, 1983, and identified there as:
(1) intermediate vector plasmid pAcgB in *Escherichia coli* K12 HB101;
(2) *Agrobacterium tumefaciens* C58C1 rifampicin-resistant strain carrying carbenicillin-resistant acceptor Ti plasmid pGV3850;
(3) intermediate vector plasmid pGV700 in *Escherichia coli* K12 strain K514 *(thr leu thi lac hsd*R);
(4) intermediate vector plasmid pGV750 in *Escherichia coli* K12 strain K514 (as above in (3));
(5) *Agrobacterium tumefaciens* C58C1 rifampicin-resistant strain carrying carbenicillin-resistant acceptor Ti plasmid pGV2260;
(6) intermediate vector plasmid pNO-1 carrying the octopine synthase coding region under the control of the nopaline promoter, in *Escherichia coli* K12 HB101;
(7) strain used in mobilization of intermediate vectors to *Agrobacterium*=GJ23 carrying mobilizing plasmids pGJ28 and R64*drd*11 (Van Haute *et al., EMBO J.* 2 (1983), 411-418); GJ23 is *Escherichia coli* K12, JC2926, a *rec*A derivative of AB1157 (Howard-Flanders *et al., Genetics* 49 (1964), 237-246).

These cultures were assigned accession numbers ... 2792 ... (1), ... 2798... (2), ... 2796... (3), ... 2797 ... (4),... 2799 ... (5),... 2833... (6), and ... 2793 ... (7), respectively.

While we have hereinbefore presented a number of embodiments of this invention, it is apparent that our basic constructions can be altered to provide other embodiments which utilize the processes and compositions of this invention.

## Claims

1. A vector combination consisting of:
i) an acceptor Ti plasmid which is substantially free of internal T-DNA sequences of a wild-type Ti plasmid and incapable of inducing tumors in plants comprising:
(a) the two border sequences (1; 2) of the T-region of a wild-type Ti plasmid;
(b) a DNA sequence (3) derived from a cloning vehicle, located between the two border sequences; and
(c) a DNA segment (4) of a wild-type Ti plasmid containing DNA sequences essential for the transfer by *Agrobacterium* of the T-region of wild-type Ti plasmids into plant cell genomes, and
ii) an intermediate cloning vector, said cloning vector comprising:
(a) at least one gene of interest (5) under the control of a promoter capable of directing gene expression in plants; and
(b) a cloning vehicle segment (3') containing a DNA sequence which is homologous with the DNA sequence (b) in said acceptor Ti plasmid permitting a single cross-over event.

2. The vector combination according to Claim 1, characterized in that the intermediate cloning vector additionally contains at least one selectable marker gene, adjacent to the gene(s) of interest (5).

3. The vector combination according to Claim 1 characterized in that the gene(s) of interest (5) contained in the intermediate cloning vector is (are) under the control of its (their) natural promoter.

4. The vector combination according to Claim 1 characterized in that the gene(s) of interest (5) contained in the intermediate cloning vector is (are) under the control of a promoter which is exogenous with respect to the gene(s) of interest.

5. A vector combination consisting of
i) an acceptor Ti plasmid, which is incapable of inducing tumors in plants being free of border sequences and internal T-DNA sequences of a wild-type Ti plasmid, comprising:
(a) a DNA segment (4) of a wild-type Ti plasmid without the T-region and without the two border sequences of the T-region; and
(b) a DNA sequence (3) derived from a cloning vehicle, and
ii) an intermediate cloning vector comprising:
(c) a cloning vehicle segment (3') containing the two border sequences of the T-region of a wild-type Ti plasmid (1; 2) and a DNA sequence located between said two border sequences which is homologous with the DNA sequence (b) in said acceptor Ti plasmid permitting a single cross-over event wherein the region between said border sequences is substantially free of internal T-DNA sequences of a wild-type Ti plasmid; and
(d) at least one gene of interest (5) under the control of a promoter capable of directing gene expression in plants located between the two border sequences in a manner allowing its integration into the plant genome.

6. The vector combination of claim 5 in which the acceptor Ti plasmid is pGV2260, DSM 2799.

7. The vector combination of claims 5 or 6 in which the intermediate cloning vector contains additionally at least one selectable marker gene adjacent to the gene(s) of interest (5).

8. The vector combination of claims 5 or 6 in which the intermediate cloning vector is further characterized in that the gene(s) of interest (5) is (are) under the control of its (their) natural promoter.

9. The vector combination of claims 5 or 6 in which the intermediate cloning vector is further characterized in that the gene(s) of interest (5) is (are) under the control of a promoter which is exogenous with respect to the gene(s) of interest.

10. The vector combination of claim 5 or 6 in which the intermediate cloning vector is pGV750, DSM 2797.

11. The acceptor Ti plasmid pGV2260, DSM 2799.

12. The intermediate cloning vector pGV750, DSM 2797.

13. A process for the preparation of a hybrid Ti plasmid which comprises producing a vector combination according to any one of Claims 1 to 4 in an *Agrobacterium* by mobilizing the intermediate cloning vector in the presence of helper plasmids to an *Agrobacterium* containing the acceptor Ti plasmid and allowing a cointegration to proceed between the intermediate cloning vector and the acceptor Ti plasmid by a single cross-over event.

14. A process for the preparation of a hybrid Ti plasmid which comprises producing a vector combination according to any one of Claims 5 to 10 in an *Agrobacterium* by mobilizing the intermediate cloning vector in the presence of helper plasmids to an *Agrobacterium* containing the acceptor Ti plasmid and allowing a cointegration to proceed between the intermediate cloning vector and the acceptor Ti plasmid by a single cross-over event.

15. A hybrid Ti plasmid vector obtained by cointegration either between an acceptor Ti plasmid which is incapable of inducing tumors in plants comprising:
(a) the two border sequences (1; 2) of the T-region of a wild-type Ti plasmid;
(b) a DNA sequence (3) devoid of the oncogenic functions of the wild-type T-DNA, derived from a cloning vehicle, located between the two border sequences, and containing a DNA sequence which is homologous with at least a part of a DNA sequence in an intermediate cloning vector permitting a single cross-over event; and
(c) a DNA segment (4) of the wild-type Ti plasmid containing DNA sequences essential for the transfer by *Agrobacterium* of the T-region of wild-type Ti plasmids into plant cell genomes; and an intermediate cloning vector comprising:
(a') at least one gene of interest (5) under the control of a promoter capable of directing gene expression in plants; and
(b') a cloning vehicle segment (3') containing a DNA sequence which is homologous with the above DNA sequence (b) in the acceptor Ti plasmid;
or between an acceptor Ti plasmid comprising:
(A) a DNA segment (4) of a wild-type Ti plasmid without the T-region and without the two border sequences of the T-region; and
(B) a DNA sequence (3) derived from a cloning vehicle; and an intermediate cloning vector comprising:
(A') a cloning vehicle segment (3') containing the two border sequences of the T-region of a wild-type Ti plasmid (1; 2) and a DNA sequence located between said two border sequences which is homologous with the above DNA sequence (B) in the acceptor Ti plasmid permitting a single cross-over event, wherein the region between said border sequences is substantially free of internal T-DNA sequences of a wild-type Ti plasmid; and
(B') at least one gene of interest (5) under the control of a promoter capable of directing gene expression in plants located between the two border sequences in a manner allowing its integration into the plant genome;
said hybrid Ti plasmid vector comprising at least:
(α) the two border sequences (1; 2) of the T-region of a wild-type Ti plasmid;
(β) non-oncogenic DNA sequences (3; 3') derived from a cloning vehicle;
(γ) a DNA segment (4) of the wild-type Ti plasmid containing DNA sequences essential for the transfer of the T-region of wild-type Ti plasmids by *Agrobacterium* into plant cell genomes; and
(δ) at least one gene of interest (5) under the control of a promoter capable of directing gene expression in plants which is located between the two border sequences (1; 2).

16. The hybrid Ti plasmid vector according to Claim 15, containing additionally at least one selectable marker gene, adjacent to the gene(s) of interest (5).

17. The hybrid Ti plasmid vector according to Claim 15 characterized in that the gene(s) of interest (5) is (are) under the control of its (their) natural promoter.

18. The hybrid Ti plasmid vector according to Claim 15, characterized in that the gene(s) of interest (5) is (are) under the control of a promoter which is exogenous with respect to the gene(s) of interest.

19. An *Agrobacterium* harboring a hybrid Ti plasmid vector according to any one of Claims 15 to 18.

20. A process for preparing a transformed plant cell comprising the infection of plant cells with an *Agrobacterium* harboring a hybrid Ti plasmid vector according to Claim 19.

21. A process for the preparation of a plant comprising the steps of:
(a) infecting a plant cell with an *Agrobacterium* according to Claim 19; and
(b) generating a plant from the transformed plant cells obtained in step (a).

## Patentansprüche

1. Vektorkombination bestehend aus:
i) einem Akzeptor-Ti-Plasmid, das im wesentlichen frei ist von internen T-DNA-Sequenzen eines Wildtyp-Ti-Plasmids und unfähig ist zur Induktion von Tumoren in Pflanzen, enthaltend:
(a) die beiden Randsequenzen (1; 2) der T-Region eines Wildtyp-Ti-Plasmids;
(b) eine DNA-Sequenz (3), die von einem Clonierungsvehikel abgeleitet ist und zwischen den beiden Randsequenzen liegt; und
(c) ein DNA-Segment (4) eines Wildtyp-Ti-Plasmids, das DNA-Sequenzen enthält, die wesentlich sind für die Übertragung der T-Region von Wildtyp-Ti-Plasmiden durch *Agrobacterium* in Pflanzenzellgenome; und
ii) einem intermediären Clonierungsvektor, wobei der Clonierungsvektor die folgenden Elemente enthält:
(a) mindestens ein gewünschtes Gen (5) unter der Kontrolle eines Promotors, der in der Lage ist, die Genexpression in Pflanzen zu steuern; und
(b) ein Clonierungsvehikel-Segment (3'), enthaltend eine DNA-Sequenz, die homolog ist zu der DNA-Sequenz (b) in dem Akzeptor-Ti-Plasmid und ein einfaches Crossover-Ereignis ermöglicht.

2. Vektorkombination nach Anspruch 1, dadurch gekennzeichnet, daß der intermediäre Clonierungsvektor zusätzlich mindestens ein Gen eines selektierbaren Markers enthält, benachbart zu dem (den) gewünschten Gen(en) (5).

3. Vektorkombination nach Anspruch 1, dadurch gekennzeichnet, daß das (die) in dem intermediären Clonierungsvektor enthaltene(n) gewünschte(n) Gen(e) (5) unter der Kontrolle seines (ihres) natürlichen Promotors steht (stehen).

4. Vektorkombination nach Anspruch 1, dadurch gekennzeichnet, daß das (die) in dem intermediären Clonierungsvektor enthaltene(n) gewünschte(n) Gen(e) (5) unter der Kontrolle eines Promotors steht (stehen), der in bezug auf das (die) gewünschte(n) Gen(e) exogen ist.

5. Vektorkombination bestehend aus
i) einem Akzeptor-Ti-Plasmid, das unfähig ist zur Induktion von Tumoren in Pflanzen und keine Randsequenzen und internen T-DNA-Sequenzen eines Wildtyp-Ti-Plasmids enthält, enthaltend:
(a) ein DNA-Segment (4) eines Wildtyp-Ti-Plasmids ohne die T-Region und ohne die beiden Randsequenzen der T-Region; und
(b) eine DNA-Sequenz (3), abgeleitet von einem Clonierungsvehikel; und
ii) einem intermediären Clonierungsvektor enthaltend:
(c) ein Clonierungsvehikel-Segment (3') enthaltend die beiden Randsequenzen der T-Region eines Wildtyp-Ti-Plasmids (1; 2) und eine DNA-Sequenz zwischen den beiden Randsequenzen, die homolog ist zu der DNA-Sequenz (b) in dem Akzeptor-Ti-Plasmid und die ein einfaches Crossover-Ereignis ermöglicht, wobei die Region zwischen den Randsequenzen im wesentlichen frei ist von internen T-DNA-Sequenzen eines Wildtyp-Ti-Plasmids; und
(d) mindestens ein gewünschtes Gen (5) unter der Kontrolle eines Promotors, der in der Lage ist, die Genexpression in Pflanzen zu steuern, das so zwischen den beiden Randsequenzen liegt, daß es in das Pflanzengenom integriert werden kann.

6. Vektorkombination nach Anspruch 5, bei der das Akzeptor-Ti-Plasmid pGV2260, DSM 2799, ist.

7. Vektorkombination nach Anspruch 5 oder 6, bei der der intermediäre Clonierungsvektor zusätzlich mindestens ein Gen eines selektierbaren Markers benachbart zu dem (den) gewünschten Gen(en) (5) enthält.

8. Vektorkombination nach Anspruch 5 oder 6, bei der der intermediäre Clonierungsvektor weiterhin dadurch gekennzeichnet ist, daß das (die) gewünschte(n) Gen(e) (5) unter der Kontrolle seines (ihres) natürlichen Promotors steht (stehen).

9. Vektorkombination nach Anspruch 5 oder 6, bei der der intermediäre Clonierungsvektor weiterhin dadurch gekennzeichnet ist, daß das (die) gewünschte(n) Gen(e) (5) unter der Kontrolle eines Promotors steht (stehen), der in bezug auf das (die) gewünschte(n) Gen(e) exogen ist.

10. Vektorkombination nach Anspruch 5 oder 6, bei der der intermediäre Clonierungsvektor pGV750, DSM 2797, ist.

11. Akzeptor-Ti-Plasmid pGV2260, DSM 2799.

12. Intermediärer Clonierungsvektor pGV750, DSM 2797.

13. Verfahren zur Herstellung eines Hybrid-Ti-Plasmids, bei dem man in einem *Agrobacterium* durch Mobilisierung des intermediären Clonierungsvektors in Gegenwart von Helferplasmiden in ein *Agrobacterium,* das das Akzeptor-Ti-Plasmid enthält, eine Vektorkombination nach einem der Ansprüche 1 bis 4 herstellt und eine Kointegration zwischen dem intermediären Clonierungsvektor und dem Akzeptor-Ti-Plasmid durch ein einfaches Crossover-Ereignis ermöglicht.

14. Verfahren zur Herstellung eines Hybrid-Ti-Plasmids, bei dem man in einem *Agrobacterium* durch Mobilisierung des intermediären Clonierungsvektors in Gegenwart von Helferplasmiden in ein *Agrobacterium,* das das Akzeptor-Ti-Plasmid enthält, eine Vektorkombination nach einem der Ansprüche 5 bis 10 herstellt und eine Kointegration zwischen dem intermediären Clonierungsvektor und dem Akzeptor-Ti-Plasmid durch ein einfaches Crossover-Ereignis ermöglicht.

15. Hybrid-Ti-Plasmidvektor erhalten durch Kointegration entweder zwischen einem Akzeptor-Ti-Plasmid, das unfähig ist zur Induktion von Tumoren in Pflanzen, enthaltend:
(a) die beiden Randsequenzen (1; 2) der T-Region eines Wildtyp-Ti-Plasmids;
(b) eine DNA-Sequenz (3) ohne die onkogenen Funktionen der Wildtyp-T-DNA, die von einem Clonierungsvehikel abgeleitet ist, zwischen den beiden Randsequenzen liegt und eine DNA-Sequenz enthält, die zu mindestens einem Teil einer DNA-Sequenz in einem intermediären Clonierungsvektor homolog ist und ein einfaches Crossover-Ereignis ermöglicht; und
(c) ein DNA-Segment (4) des Wildtyp-Ti-Plasmids, das DNA-Sequenzen enthält, die wesentlich sind für die Übertragung der T-Region von Wildtyp-Ti-Plasmiden durch *Agrobacterium* in Pflanzenzellgenome;
und einem intermediären Clonierungsvektor enthaltend:
(a') mindestens ein gewünschtes Gen (5) unter der Kontrolle eines Promotors, der in der Lage ist, die Genexpression in Pflanzen zu steuern; und
(b') ein Clonierungsvehikel-Segment (3'), das eine DNA-Sequenz enthält, die homolog ist zu der obengenannten DNA-Sequenz (b) in dem Akzeptor-Ti-Plasmid;
oder zwischen einem Akzeptor-Ti-Plasmid enthaltend:
(A) ein DNA-Segment (4) eines Wildtyp-Ti-Plasmids ohne die T-Region und ohne die beiden Randsequenzen der T-Region; und
(B) eine DNA-Sequenz (3) abgeleitet von einem Clonierungsvehikel;
und einem intermediären Clonierungsvektor enthaltend:
(A') ein Clonierungsvehikel-Segment (3'), das die beiden Randsequenzen der T-Region eines Wildtyp-Ti-Plasmids (1; 2) enthält und eine DNA-Sequenz, die zwischen den beiden Randsequenzen lokalisiert ist, die homolog ist zu der obengenannten DNA-Sequenz (B) in dem Akzeptor-Ti-Plasmid und die ein einfaches Crossover-Ereignis ermöglicht, wobei die Region zwischen den Randsequenzen im wesentlichen frei ist von internen T-DNA-Sequenzen eines Wildtyp-Ti-Plasmids; und
(B') mindestens ein gewünschtes Gen (5) unter der Kontrolle eines Promotors, der in der Lage ist, die Genexpression in Pflanzen zu steuern, und das so zwischen den beiden Randsequenzen liegt, daß es in das Pflanzengenom integriert werden kann;
wobei der Hybrid-Ti-Plasmidvektor mindestens folgende Elemente enthält:
(α) die beiden Randsequenzen (1; 2) der T-Region eines Wildtyp-Ti-Plasmids;
(β) nicht-onkogene DNA-Sequenzen (3; 3') abgeleitet von einem Clonierungsvehikel;
(γ) ein DNA-Segment (4) des Wildtyp-Ti-Plasmids, das DNA-Sequenzen enthält, die wesentlich sind für die Übertragung der T-Region von Wildtyp-Ti-Plasmiden durch *Agrobacterium* in Pflanzenzellgenome; und
(δ) mindestens ein gewünschtes Gen (5) unter der Kontrolle eines Promotors, der in der Lage ist, die Genexpression in Pflanzen zu steuern, und das zwischen den beiden Randsequenzen (1; 2) liegt.

16. Hybrid-Ti-Plasmidvektor nach Anspruch 15, zusätzlich enthaltend mindestens ein Gen für einen selektierbaren Marker, das benachbart zu dem (den) gewünschten Gen(en) (5) liegt.

17. Hybrid-Ti-Plasmidvektor nach Anspruch 15, dadurch gekennzeichnet, daß das (die) gewünschte(n) Gen(e) (5) unter der Kontrolle seines (ihres) natürlichen Promotors steht (stehen).

18. Hybrid-Ti-Plasmidvektor nach Anspruch 15, dadurch gekennzeichnet, daß das (die) gewünschte(n) Gen(e) (5) unter der Kontrolle eines Promotors steht (stehen), der in bezug auf das (die) gewünschte(n) Gen(e) exogen ist.

19. *Agrobacterium* enthaltend einen Hybrid-Ti-Plasmidvektor nach einem der Ansprüche 15 bis 18.

20. Verfahren zur Herstellung einer transformierten Pflanzenzelle, bei dem man Pflanzenzellen mit einem *Agrobacterium* nach Anspruch 19 infiziert, das einen Hybrid-Ti-Plasmidvektor enthält.

21. Verfahren zur Herstellung einer Pflanze, bei dem man:
(a) eine Pflanzenzelle mit einem *Agrobacterium* nach Anspruch 19 infiziert; und
(b) eine Pflanze aus den gemäß Stufe (a) erhaltenen transformierten Pflanzenzellen erzeugt.

## Revendications

1. Combinaison vectorielle consistant en :
i) un plasmide Ti accepteur qui est essentiellement exempt de séquences d'ADN T internes d'un plasmide Ti du type sauvage et incapable d'induire des tumeurs chez les plantes comprenant :
(a) les deux séquences bordures (1; 2) de la région T d'un plasmide Ti du type sauvage;
(b) une séquence d'ADN (3) provenant d'un véhicule de clonage, localisée entre les deux séquences bordures; et
(c) un segment d'ADN (4) d'un plasmide Ti du type sauvage contenant des séquences d'ADN essentielles au transfert par Agrobacterium de la région T de plasmides Ti du type sauvage dans des génomes de cellules végétales, et
ii) un vecteur de clonage intermédiaire, ledit vecteur de clonage comprenant :
(a) au moins un gène d'intérêt (5) sous le contrôle d'un promoteur pouvant diriger l'expression des gènes chez les plantes; et
(b) un segment de véhicule de clonage (3') contenant une séquence d'ADN qui est homologue à la séquence d'ADN (b) dans le plasmide Ti accepteur permettant un cas de simple cross-over.

2. Combinaison vectorielle suivant la revendication 1, caractérisée en ce que le vecteur de clonage intermédiaire contient de plus au moins un gène marqueur sélectionnable, adjacent au(x) gène(s) d'intérêt (5).

3. Combinaison vectorielle suivant la revendication 1, caractérisée en ce que le(s) gène(s) d'intérêt (5) contenu(s) dans le vecteur de clonage intermédiaire est (sont) sous le contrôle de son (leur) promoteur naturel.

4. Combinaison vectorielle suivant la revendication 1, caractérisée en ce que le(s) gène(s) d'intérêt (5) contenu(s) dans le vecteur de clonage intermédiaire est (sont) sous le contrôle d'un promoteur qui est hexogène par rapport au(x) gène(s) d'intérêt.

5. Combinaison vectorielle consistant en
i) un plasmide Ti accepteur, qui est incapable d'induire des tumeurs chez les plantes étant exempt de séquences bordures et de séquences d'ADN T internes d'un plasmide Ti du type sauvage, comprenant :
(a) un segment d'ADN (4) d'un plasmide Ti du type sauvage sans la région T et sans les deux séquences bordures de la région T; et
(b) une séquence d'ADN (3) provenant d'un véhicule de clonage, et
ii) un vecteur de clonage intermédiaire comprenant :
(c) un segment de véhicule de clonage (3') contenant les deux séquences bordures de la région T d'un plasmide Ti du type sauvage (1; 2) et une séquence d'ADN localisée entre les deux séquences bordures qui est homologue à la séquence d'ADN (b) dans le plasmide Ti accepteur permettant un cas de simple cross-over, dans lequel la région entre les séquences bordures est essentiellement exempte de séquences d'ADN T internes d'un plasmide Ti du type sauvage; et
(d) au moins un gène d'intérêt (5) sous le contrôle d'un promoteur pouvant diriger l'expression des gènes chez les plantes localisé entre les deux séquences bordures d'une manière permettant son intégration dans le génome des plantes.

6. Combinaison vectorielle suivant la revendication 5, dans laquelle le plasmide Ti accepteur est le pGV2260, DSM 2799.

7. Combinaison vectorielle suivant l'une ou l'autre des revendications 5 et 6, dans laquelle le vecteur de clonage intermédiaire contient de plus au moins un gène marqueur sélectionnable adjacent au(x) gène(s) d'intérêt (5).

8. Combinaison vectorielle suivant l'une ou l'autre des revendications 5 et 6, dans laquelle le vecteur de clonage intermédiaire est de plus caractérisé en ce que le(s) gène(s) d'intérêt (5) est (sont) sous le contrôle de son (leur) promoteur naturel.

9. Combinaison vectorielle suivant l'une ou l'autre des revendications 5 et 6, dans laquelle le vecteur de clonage intermédiaire est de plus caractérisé en ce que le(s) gène(s) d'intérêt (5) est (sont) sous le contrôle d'un promoteur qui est exogène par rapport au(x) gène(s) d'intérêt.

10. Combinaison vectorielle suivant l'une ou l'autre des revendications 5 et 6, dans laquelle le vecteur de clonage intermédiaire est le pGV750, DSM 2797.

11. Le plasmide Ti accepteur pGV2260, DSM 2799.

12. Le vecteur de clonage intermédiaire pGV750, DSM 2797.

13. Procédé de préparation d'un plasmide Ti hybride qui comprend la production d'une combinaison vectorielle suivant l'une quelconque des revendications 1 à 4 dans un Agrobacterium en mobilisant le vecteur de clonage intermédiaire en présence de plasmides aidants vers un Agrobacterium contenant le plasmide Ti accepteur et en laissant une cointégration se produire entre le vecteur de clonage intermédiaire et le plasmide Ti accepteur par un cas de cross-over simple.

14. Procédé de préparation d'un plasmide Ti hybride qui comprend la production d'une combinaison vectorielle suivant l'une quelconque des revendications 5 à 10 dans un Agrobacterium en mobilisant le vecteur de clonage intermédiaire en présence de plasmides aidants vers un Agrobacterium contenant le plasmide Ti accepteur et en laissant une cointégration se produire entre le vecteur de clonage intermédiaire et le plasmide Ti accepteur par un cas de cross-over simple.

15. Vecteur plasmidique Ti hybride obtenu par cointégration soit entre un plasmide Ti accepteur qui est incapable d'induire des tumeurs chez les plantes comprenant :
(a) les deux séquences bordures (1; 2) de la région T d'un plasmide Ti du type sauvage;
(b) une séquence d'ADN (3) dépourvue des fonctions oncogéniques de l'ADN T du type sauvage, provenant d'un véhicule de clonage, localisée entre les deux séquences bordures, et contenant une séquence d'ADN qui est homologue à au moins une partie d'une séquence d'ADN dans un vecteur de clonage intermédiaire, permettant un cas de simple cross-over; et
(c) un segment d'ADN (4) du plasmide Ti du type sauvage contenant des séquences d'ADN essentielles au transfert par Agrobacterium de la région T de plasmides Ti du type sauvage dans des génomes de cellules végétales; et un vecteur de clonage intermédiaire comprenant :
(a') au moins un gène d'intérêt (5) sous le contrôle d'un promoteur pouvant diriger l'expression des gènes chez les plantes; et
(b') un segment de véhicule de clonage (3') contenant une séquence d'ADN qui est homologue à la séquence d'ADN (b) susmentionnée dans le plasmide Ti accepteur;
soit entre un plasmide Ti accepteur comprenant :
(A) un segment d'ADN (4) d'un plasmide Ti du type sauvage sans la région T et sans les deux séquences bordures de la région T; et
(B) une séquence d'ADN (3) provenant d'un véhicule de clonage; et un vecteur de clonage intermédiaire comprenant :
(A') un segment de véhicule de clonage (3') contenant les deux séquences bordures de la région T d'un plasmide Ti de type sauvage (1; 2) et une séquence d'ADN localisée entre les deux séquences bordures qui est homologue à la séquence d'ADN (B) susmentionnée dans le plasmide Ti accepteur permettant un cas de simple cross-over, dans lequel la région entre les séquences bordures est essentiellement exempte de séquences d'ADN T internes d'un plasmide Ti du type sauvage; et
(B') au moins un gène d'intérêt (5) sous le contrôle d'un promoteur pouvant diriger l'expression des gènes chez les plantes localisé entre les deux séquences bordures d'une manière permettant son intégration dans le génome des plantes;
le vecteur plasmidique Ti hybride comprenant au moins :
(α) les deux séquences bordures (1; 2) de la région T d'un plasmide Ti du type sauvage;
(β) des séquences d'ADN non oncogéniques (3, 3') provenant d'un véhicule de clonage;
(γ) un segment d'ADN (4) du plasmide Ti du type sauvage contenant des séquences d'ADN essentielles au transfert de la région T de plasmides Ti du type sauvage par Agrobacterium dans des génomes de cellules végétales; et
(δ) au moins un gène d'intérêt (5) sous le contrôle d'un promoteur pouvant diriger l'expression des gènes chez les plantes qui est localisé entre les deux séquences bordures (1; 2).

16. Vecteur plasmidique Ti hybride suivant la revendication 15, contenant de plus au moins un gène marqueur sélectionnable, adjacent au(x) gène(s) d'intérêt (5).

17. Vecteur plasmidique Ti hybride suivant la revendication 15, caractérisé en ce que le(s) gène(s) d'intérêt (5) est (sont) sous le contrôle de son (leur) promoteur naturel.

18. Vecteur plasmidique Ti hybride suivant la revendication 15, caractérisé en ce que le(s) gène(s) d'intérêt (5) est (sont) sous le contrôle d'un promoteur qui est hexogène par rapport au(x) gène(s) d'intérêt.

19. Agrobacterium abritant un vecteur plasmidique Ti hybride suivant l'une quelconque des revendications 15 à 18.

20. Procédé de préparation d'une cellule végétale transformée comprenant l'infection de cellules végétales par un Agrobacterium abritant un vecteur plasmidique Ti hybride suivant la revendication 19.

21. Procédé de préparation d'une plante comprenant les étapes :
(a) d'infection d'une cellule végétale par un Agrobacterium suivant la revendication 19; et
(b) de production d'une plante à partir des cellules végétales transformées obtenues dans l'étape (a).
